# EUROPÄISCHE PATENTSCHRIFT

(11) **EP 1 019 368 B1**
(45) Veröffentlichungstag und Bekanntmachung des Hinweises auf die Patenterteilung: **05.03.2003**
(21) Anmeldenummer: 98952644.7
(22) Anmeldetag: 24.09.1998
(51) Int. Cl.: C07C 311/51, C07D 213/81, A01N 37/28, A01N 43/40

(54) **ACYLSULFAMOYLBENZOESÄUREAMIDE, DIESE ENTHALTENDE NUTZPFLANZENSCHÜTZENDE MITTEL UND VERFAHREN ZU IHRER HERSTELLUNG**
ACYLSULFAMOYL BENZOIC ACID AMIDES, PLANT PROTECTION AGENTS CONTAINING SAID ACYLSULFAMOYL BENZOIC ACID AMIDES, AND METHOD FOR PRODUCING THE SAME
AMIDES D'ACIDE BENZOIQUE D'ACYLSULFAMOYLE, AGENTS PHYTOSANITAIRES LES CONTENANT ET PROCEDE PERMETTANT DE LES PREPARER

(30) Priorität: 29.09.1997 DE 19742951
(43) Veröffentlichungstag der Anmeldung: 19.07.2000
(73) Patentinhaber: Bayer CropScience GmbH, 65929 Frankfurt/Main (DE)
(72) Erfinder: ZIEMER, Frank, D-65830 Kriftel (DE); WILLMS, Lothar, D-65719 Hofheim (DE); AULER, Thomas, D-65812 Bad Soden (DE); BIERINGER, Hermann, D-65817 Eppstein (DE); ROSINGER, Christopher, D-65719 Hofheim (DE)
(86) Internationale Anmeldenummer: EP9806097
(87) Internationale Veröffentlichungsnummer: WO99016744

(56) Entgegenhaltungen:
- EP-A- 0 562 512
- EP-A- 0 567 997
- EP-A- 0 590 520
- EP-A- 0 673 932
- WO-A-96/10559
- DE-A- 19 621 522
- PATENT ABSTRACTS OF JAPAN vol. 009, no. 134 (C-285), 8. Juni 1985 & JP 60 019761 A (MEIJI SEIKA KK), 31. Januar 1985
- CHEMICAL ABSTRACTS, vol. 84, no. 3, 19. Januar 1976 Columbus, Ohio, US; abstract no. 17204b, T. TOKUMITSU, ET AL.: "Reaction of alpha,beta-unsaturated beta-amino ketones with thiocyanogen" XP002092978 in der Anmeldung erwähnt -& CHEMICAL ABSTRACTS, 9TH COLLECTIVE CHEMICAL SUBSTANCES INDEX, Seite 2905 XP002092976 -& CHEMICAL ABSTRACTS, 9TH COLLECTIVE CHEMICAL SUBSTANCES INDEX, Seite 6364 XP002092977 & NIPPON KAGAKU KAISHI, Nr. 1, 1975, Seiten 123-126,

## Beschreibung

Die vorliegende Erfindung betrifft nutzpflanzenschützende Mittel, die Acylsulfamoylbenzoesäureamide und gegebenenfalls Pestizide enthalten, sowie bestimmte Acylsulfamoylbenzoesäureamide und Verfahren zu ihrer Herstellung.

Bei der Bekämpfung unerwünschter Organismen in Land- und forstwirtschaftlichen Nutzpflanzenkulturen mit Pestiziden werden - in an sich unerwünschter Weise - häufig auch die Nutzpflanzen durch die verwendeten Pestizide mehr oder weniger stark geschädigt. Dieser Effekt tritt in besonderem Maße bei der Verwendung von zahlreichen Herbiziden - und dort in erster Linie bei der sogenannten Nachauflaufapplikation - in Nutzpflanzenkulturen wie Mais, Reis oder Getreide auf. Durch den Einsatz sogenannter "Safener" oder "Antidots" können in manchen Fällen die Nutzpflanzen gegen die phytotoxischen Eigenschaften der Pestizide geschützt werden, ohne daß die pestizide Wirkung gegenüber den Schadorganismen geschmälert wird.

Die bislang als Safener bekannt gewordenen Verbindungen weisen unterschiedliche chemische Strukturen auf. So sind aus US-A 4,902,340 Derivate von Chinolin-8-oxy-alkancarbonsäuren als Safener für Herbizide aus der Reihe der Diphenylether und der Pyridyloxyphenoxypropionsäuren und aus EP-A 0 520 371 Isoxazoline sowie Isothiazoline als Safener für verschiedene Arten von Herbiziden bekannt, wobei in der letztgenannten Veröffentlichung Aryloxyphenoxycarbonsäuren, Sulfonylharnstoffe und Imidazolinone als bevorzugte Herbizide genannt sind.

Imai et al. beschreiben in *Nippon Kagaku Kaishi,* **1975**, 123-126, (Chem. *Abstr.,* (1976), **84**: 17204) drei Acylsulfamoylbenzoesäureamide, speziell die Verbindungen
- 2-(Benzoylsulfamoyl)-N-phenylbenzamid
- 2-(Benzoylsulfamoyl)-N-benzylbenzamid
- 4-(2-Benzoylsulfamoyl-benzoylamino)-benzoesäure.

Eine besondere biologische Wirkung oder andere Eigenschaften dieser Verbindungen sind nicht genannt.

In EP-A 0 562 512 werden Acylsulfamoylbenzoesäureamide beschrieben, die in 2-bzw. in 6-Position des Pyridinrings eine Aminocarbonylgruppe tragen. Aus EP-A 0 590 520 sind Acylsulfamoylbenzoesäureamide bekannt, die in 2- bzw. in 6-Position des Pyridinrings gegebenenfalls durch eine Estergruppe substituiert sind. EP-A 0 673 932 nennt Acylsulfamoylbenzoesäureamide, die in 2- und 4-Position des Pyridinrings jeweils eine Aminocarbonylgruppe tragen. Die in diesen drei Veröffentlichungen genannten Acylsulfamoylbenzoesäureamide werden als Arzneimittel gegen fibrotische Erkrankungen beschrieben. Eine Safener-Wirkung von Acylsulf-amoylbenzoesäureamiden ist bislang nicht bekannt. Aus EP-A 0 567 997 sind einige Acylsulfamoylpyridincarbonsäuren mit Eigenschaften gegen gegen fibrotische Erkrankungen bekannt.

Bei der Anwendung von Safenern zum Schutz der Nutzpflanzen vor den Pestizidschädigungen hat sich gezeigt, daß die bekannten Safener in vielen Fällen immer noch gewisse Nachteile aufweisen können. Dazu zählen:
- der Safener vermindert die Wirkung der Pestizide, insbesondere die von Herbiziden, gegen die Schadpflanzen
- die nutzpflanzenschützenden Eigenschaften sind nicht ausreichend
- in Kombination mit einem gegebenen Herbizid ist das Spektrum der Nutzpflanzen, in denen der Safener/Herbizid-Einsatz erfolgen soll, nicht ausreichend groß
- ein gegebener Safener ist nicht mit einer ausreichend großen Anzahl von Herbiziden kombinierbar.

Aufgabe der vorliegenden Erfindung ist die Bereitstellung nutzpflanzenschützender Mittel, die Verbindungen mit verbessenter Safener-Wirkung und gegebenenfalls Pestizide enthalten.

Die Lösung der Aufgabe sind gegebenenfalls mindestens ein Pestizid enthaltende nutzpflanzenschützende Mittel, gekennzeichnet durch einen Gehalt mindestens eines Acylsulfamoylbenzoesäureamids der allgemeinen Formel l, gegebenenfalls auch in seiner Salzform, 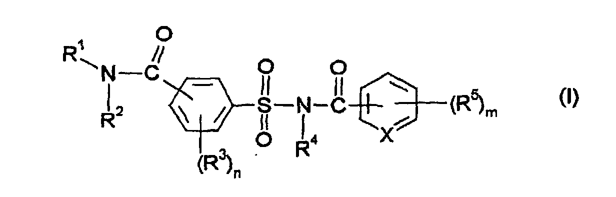 in der
X CH oder N;
R¹ Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
R² Wasserstoff, Hydroxy, (C₁-C₆)-Alkyl, (C₂-C₆)-Alkenyl, (C₂-C₆)-Alkinyl, (C₁-C₆)-Alkoxy, (C₂-C₆)-Alkenyloxy, wobei die fünf letztgenannten Reste gegebenenfalls durch einen oder mehrere, gleiche oder verschiedene Reste aus der Gruppe Halogen, Hydroxy, (C₁-C₄)-Alkyl, (C₁-C₄)-Alkoxy und (C₁-C₄)-Alkylthio substituiert sind, oder
R¹ und R² zusammen mit dem sie tragenden Stickstoffatom einen 3- bis 8-gliedrigen gesättigten oder ungesättigten Ring bilden;
R³ Halogen, Cyano, Nitro, Amino, Hydroxy, Carboxy, CHO, CONH₂, SO₂NH₂ oder Z^{b}-R^{b};
R⁴ Wasserstoff, (C₁-C₄)-Alkyl, (C₂-C₄)-Alkenyl oder (C₂-C₄)-Alkinyl;
R⁵ Halogen, Cyano, Nitro, Amino, Hydroxy, Phosphoryl, CHO, CONH₂, SO₂NH₂ oder Z^{c}-R^{c};
R^{a} einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
R^{b}, R^{c} unabhängig voneinander einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
Z^{a} eine divalente Einheit aus der Gruppe O, S, CO, CS, C(O)O, C(O)S, SO, SO₂, NR^{d}, C(O)NR^{d} oder SC₂NR^{d};
Z^{b}, Z^{c} unabhängig voneinander eine direkte Bindung oder eine divalente Einheit aus der Gruppe O, S, CO, CS, C(O)O, C(O)S, SO, SO₂, NR^{d}, SO₂NR^{d} oder C(O)NR^{d};
R^{d} Wasserstoff, (C₁-C₄)-Alkyl oder (C₁-C₄)-Haloalkyl;
n eine ganze Zahl von 0 bis 4
   und
m für den Fall, daß X für CH steht, eine ganze Zahl von 0 bis 5, und für den Fall, daß X für N steht, eine ganze Zahl von 0 bis 4
bedeuten.

Die vorstehend und weiter unter verwendeten Bezeichnungen haben die im folgenden erläuterten Bedeutungen:

Die Bezeichnung "Halogen" umfaßt Fluor, Chlor, Brom und Jod. Unter dem Ausdruck "(C₁-C₄)-Alkyl" ist ein unverzweigter oder verzweigter Kohlenwasserstoffrest mit 1, 2, 3 oder 4 Kohlenstoffatomen, z.B. der Methyl-, Ethyl-, Propyl-, Isopropyl-, 1-Butyl-, 2-Butyl-, 2-Methylpropyl- oder tert.-Butylrest zu verstehen. Entsprechend ist unter Alkylresten mit einem größeren Bereich an Kohlenstoffatomen ein unverzweigter oder verzweigter gesättigter Kohlenwasserstoffrest zu verstehen, der eine Anzahl an Kohlenstoffatomen enthält, die dieser Bereichsangabe entspricht. Der Ausdruck "(C₁-C₆)-Alkyl" umfaßt demnach die vorgenannten Alkylreste, sowie z.B. den Pentyl-, 2-Methylbutyl-, 1,1-Dimethylpropyl- und Hexyl-Rest.

Ist die Kohlenstoffkette eines Alkylrests mehrfach durch Sauerstoffatome unterbrochen, so bedeutet dies, daß zwei Sauerstoffatome niemals direkt benachbart sein sollen.

Unter "(C₁-C₄)-Haloalkyl" ist eine unter dem Ausdruck "(C₁-C₄)-Alkyl" genannte Alkylgruppe zu verstehen, in der eines oder mehrere Wasserstoffatome durch die entsprechende Anzahl gleicher oder verschiedener Halogenatome, bevorzugt Chlor oder Fluor, ersetzt sind, wie die Trifluormethyl-, die 1-Fluorethyl-, die 2,2,2-Trifluorethyl-, die Chlormethyl-, Fluormethyl-, die Difluormethyl- und die 1,1,2,2-Tetrafluorethylgruppe.
Unter "(C₁-C₄)-Alkoxy" ist eine Alkoxygruppe zu verstehen, deren Kohlenwasserstoffrest die unter dem Ausdruck "(C₁-C₄)-Alkyl" angegebene Bedeutung hat. Sinngemäß sind auch solche Alkoxygruppen zu verstehen, die einen größeren Bereich an Kohlenstoffatomen umfassen.
Die Bezeichnungen "Alkenyl" und "Alkinyl" mit einer vorangestellten Bereichsangabe von Kohlenstoffatomen bedeuten einen geradkettigen oder verzweigten Kohlenwasserstoffrest mit einer dieser Bereichsangabe entsprechenden Kohlenstoff-atomzahl, wobei dieser Kohlenwasserstoffrest mindestens eine Mehrfachbindung beinhaltet, und sich diese an beliebiger Position des betreffenden ungesättigten Restes befinden kann. "(C₂-C₆)-Alkenyl" steht demnach z.B. für die Vinyl-, Allyl-, 2-Methyl-2-propenyl-, 2-Butenyl-, Pentenyl-, 2-Methylpentenyl- oder die Hexenyl-Gruppe. "(C₂-C₆)-Alkinyl" steht z.B. für die Ethinyl-, Propargyl-, 2-Methyl-2-propinyl-, 2-Butinyl-, 2-Pentinyl- und die 2-Hexinyl-Gruppe.
"(C₃-C₈)-Cycloalkyl" steht für monocyclische Alkylreste, wie den Cyclopropyl-, Cyclobutyl-, Cyclopentyl-, Cyclohexyl-, Cycloheptyl- oder Cyclooctylrest und für bicyclische Alkylreste, wie den Norbornylrest.
Unter "(C₃-C₈)-Cycloalkoxy" oder "(C₃-C₈)-Cycloalkylthio" ist einer der oben angeführten (C₃-C₈)-Cycloalkyl-Reste, der über ein Sauerstoff- oder Schwefelatom verknüpft ist, zu verstehen.

"(C₁-C₆)-Alkylthio" steht für eine Alkylthiogruppe, deren Kohlenwasserstoffrest die unter dem Ausdruck "(C₁-C₆)-Alkyl" angegebene Bedeutung hat. Analog bedeuten "(C₁-C₈)-Alkylsulfinyl" z.B. die Methyl-, Ethyl-, Propyl-, Isopropyl-, Butyl-, Isobutyl-, sek.-Butyl- oder tert.-Butylsulfinyl-Gruppe und "(C₁-C₆)-Alkylsulfonyl" z.B. die Methyl-, Ethyl-, Propyl-, Isopropyl-, Butyl-, Isobutyl-, sek.-Butyl- und tert.-Butylsulfonyl-Gruppe.
"Mono- und Di-[(C₁-C₄)-alkyl]-amino" steht für ein Stickstoffatom, das durch ein oder zwei, gleiche oder verschiedene Alkylreste der obigen Definition substituiert ist. Analog oben stehender Definitionen sind sinngemäß andere zusammengesetzte Bezeichnungen, wie (C₃-C₆)-Cycloalkenyl und [(C₁-C₆)-(Alkylthio]-carbonyl zu verstehen.

Unter dem Ausdruck "Aryl" ist ein isocyclischer, mono-, bi- oder polycyclischer aromatischer Rest mit vorzugsweise 6 bis 14, insbesondere 6 bis 12 C-Atomen, wie Phenyl, Naphthyl oder Biphenylyl, vorzugsweise Phenyl zu verstehen.
Der Ausdruck "Heterocyclyl" steht für einen mono- oder bicyclischen Rest, der vollständig gesättigt, teilweise oder vollständig ungesättigt ist und der ein bis fünf gleiche oder verschiedene Atome aus der Gruppe Stickstoff, Schwefel und Sauerstoff enthält, wobei jedoch nicht zwei Sauerstoffatome direkt benachbart sein dürfen und noch mindestens ein Kohlenstoffatom im Ring vorhanden sein muß, z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, Piperidin, Pyrrolidin, Oxazolin, Tetrahydrofuran, Tetrahydropyran, Isoxazolidin oder Thiazolidin.

Ein "Kohlenwasserstoffrest" ist ein geradkettiger, verzweigter oder cyclischer Kohlenwasserstoffrest, der gesättigt, teilgesättigt, ungesättigt oder aromatisch sein kann, z.B. Alkyl, Alkenyl, Alkinyl, Cycloalkyl, Cycloalkenyl und Aryl, vorzugsweise Alkyl, Alkenyl und Alkinyl mit bis zu 20 C-Atomen oder Cycloalkyl mit 3 bis 6 Ringatomen oder Phenyl.
In den Fällen, in denen zwei oder mehrere Reste R³ und/oder R⁵ an einem Benzoloder Pyridinring auftreten, d. h. wenn m und/oder n größer eins sind, können diese Reste jeweils gleich oder verschieden sein.

Bedeutet R¹ in der allgemeinen Formel l einen Kohlenwasserstoffrest, so hat dieser vorzugsweise bis zu 20 Kohlenstoffatome. Trägt dieser Kohlenwasserstoffrest noch weitere kohlenstoffhaltige Substituenten, so ist die Gesamtzahl aller Kohlenstoffatome dieses Rests R¹ vorzugsweise 2 bis 30.
Für den Fall daß Z^{a}, Z^{b} und/oder Z^{c} eine divalente Einheit bedeuten, die unsymmetrisch ist, d.h. daß sie zwei Möglichkeiten der Verknüpfung erlaubt, sind jeweils beide Möglichkeiten der Verknüpfung von Z^{a}, Z^{b}, Z^{c} mit R^{a}, R^{b}, R^{c} einerseits und dem Rest des Moleküls andererseits durch die allgemeine Formel l umfaßt.

Die Verbindungen der allgemeinen Formel l können je nach Art und Verknüpfung der Substituenten als Stereoisomere vorliegen. Sind beispielsweise eine oder mehrere Alkenylgruppen vorhanden, so können Diastereomere auftreten. Sind beispielsweise ein oder mehrere asymmetrische Kohlenstoffatome vorhanden, so können Enantiomere und Diastereomere auftreten. Stereoisomere lassen sich aus Die Kombinationsmöglichkeiten der verschiedenen Substituenten der allgemeinen Formel l sind so zu verstehen, daß die allgemeinen Grundsätze des Aufbaus chemischer Verbindungen zu beachten sind, d.h. daß nicht Verbindungen gebildet werden sollen, von denen der Fachmann weiß, daß sie chemisch instabil oder nicht möglich sind.
Die Verbindungen der Formel l können Salze bilden. Salzbildung kann durch Einwirkung einer Base auf solche Verbindungen der Formel l erfolgen, die ein acides Wasserstoffatom tragen, z.B. im Falle von R⁴ = H oder R⁵ = COOH.
Geeignete Basen sind beispielsweise organische Amine sowie Ammonium-, Alkalioder Erdalkalimetallhydroxide, -carbonate und -hydrogencarbonate, insbesondere Natrium- und Kaliumhydroxid, -carbonat und -hydrogencarbonat. Ebenso kann Salzbildung durch Anlagerung einer Säure an basische Gruppen, wie Amino und Alkylamino, erfolgen. Geeignete Säuren hierfür sind anorganische und organische Säuren, beispielsweise HCI, HBr, H₂SO₄, HNO₃ und Essigsäure.

In den nutzpflanzenschützenden Mitteln sind solche Verbindungen der allgemeinen Formel l von näherem Interesse, in der
R¹ Wasserstoff, 3- bis 8-gliedriges Heterocyclyl mit bis zu drei gleichen oder verschiedenen Heteroatomen aus der Gruppe Stickstoff, Sauerstoff und Schwefel, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl oder Aryl, wobei die sieben letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
R⁵ Halogen, Cyano, Nitro, Amino, Hydroxy, CHO, CONH₂, SO₂NH₂ oder Z^{c}-R^{c}.
R^{a} einen (C₂-C₁₂)-Alkylrest, dessen Kohlenstoffkette ein- oder mehrfach durch Sauerstoffatome unterbrochen ist, (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder 3- bis 6-gliedriges Heterocyclyl mit bis zu drei gleichen oder verschiedenen Heteroatomen aus der Gruppe Stickstoff, Sauerstoff und Schwefel, wobei die sieben letztgenannten Reste unabhängig voneinander gegebenenfalls durch einen oder mehrere, gleiche oder verschiedene Reste aus der Gruppe Halogen, Cyano, Nitro, Amino, Hydroxy, Monound Di-[(C₁-C₄)-alkyl]-amino substituiert sind;
R^{b}, R^{c} unabhängig voneinander einen (C₂-C₁₂)-Alkylrest, dessen Kohlenstoffkette ein- oder mehrfach durch Sauerstoffatome unterbrochen ist, (C₁-C₈)-Alkyl, (C₃-C₆)-Cycloalkyl, (C₂-C₈)-Alkenyl, (C₃-C₆)-Cycloalkenyl, (C₂-C₈)-AIkinyl, Phenyl oder 3- bis 6-gliedriges Heterocyclyl mit bis zu drei gleichen oder verschiedenen Heteroatomen aus der Gruppe N, O und S, wobei die sieben letztgenannten Reste unabhäng-ig voneinander gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
Z^{a} eine divalente Einheit aus der Gruppe O, S, CO, CS, C(O)O, C(O)S, SO, SO₂, NR^{d} oder C(O)NR^{d};
Z^{b}, Z^{c} unabhängig voneinander eine direkte Bindung oder eine divalente Einheit aus der Gruppe O, S, CO, CS, C(O)O, C(O)S, SO, SO₂, NR^{d}, SOₐNR^{d} oder C(O)NR^{d} und
R^{d} Wasserstoff, (C₁-C₄)-Alkyl oder (C₁-C₄)-Haloalkyl
bedeuten.

Von besonderem Interesse sind in den nutzpflanzenschützenden Mitteln solche Verbindungen der allgemeinen Formel l, in der
R¹ Wasserstoff, (C₁-C₈)-Alkyl, (C₂-C₆)-Alkenyl, (C₂-C₆)-Alkinyl, (C₃-C₆)-Cycloalkyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sieben letztgenannten Reste gegebenenfalls durch einen oder mehrere, gleiche oder verschiedene Substituenten aus der Gruppe Halogen, Cyano, (C₁-C₈)-Alkoxy, (C₁-C₆)-Alkylthio, (C₁-C₄)-Alkylsulfinyl, (C₁-C₄)-Alkylsulfonyl, (C₂-C₄)-Alkenyloxy, (C₂-C₄)-Alkenylthio, (C₂-C₄)-Alkinyloxy, (C₂-C₄)-Alkinylthio, (C₃-C₆)-Cycloalkyl, (C₅-C₆)-Cycloalkenyl, (C₃-C₆)-Cycloalkoxy, (C₅-C₆)-Cycloalkenyloxy, Mono- und Di-[(C₁-C₄)-alkyl]-amino, [(C₁-C₆)-Alkoxy]-carbonyl, [(C₁-C₆)-Alkylthio]-carbonyl, [(C₁-C₆)-Alkyl]-carbonyl, Phenyl, Phenyl-(C₁-C₄)-alkoxy, 5- bis 6-gliedriges Heterocyclyl mit bis zu drei Heteroatomen aus der Gruppe Stickstoff, Sauerstoff und Schwefel und im Falle cyclischer Reste auch (C₁-C₄)-Alkyl substituiert sind, wobei die zwanzig letztgenannten Reste gegebenenfalls durch einen oder mehrere, gleiche oder verschiedene Substituenten aus der Gruppe Halogen und Cyano und im Falle cyclischer Reste auch (C₁-C₄)-Alkyl substituiert sind;
R³, R⁵ unabhängig voneinander Halogen, Nitro, Amino, Hydroxy, Cyano, SO₂NH₂, (C₁-C₆)-Alkyl, (C₃-C₆)-Cycloalkyl, (C₂-C₆)-Alkenyl, (C₂-C₆)-Alkinyl, (C₁-C₆)-Alkoxy, (C₂-C₆)-Alkenytoxy, (C₂-C₆)-Alkinyloxy, Mono- und Di-[(C₁-C₄)-Alkyl]-aminosulfonyl, (C₁-C₆)-Alkylthio, (C₁-C₈)-Alkylsulfinyl, (C₁-C₆)-Alkylsulfonyl, (C₁-C₆)-Alkoxycarbonyl, (C₁-C₆)-Alkylthiocarbonyl, (C₁-C₆)-Alkylcarbonyl, wobei die fünfzehn letztgenannten Reste gegebenenfalls durch einen oder mehrere, gleiche oder verschiedene Reste aus der Gruppe Halogen, (C₁-C₄)-Haloalkoxy, Cyano, (C₁-C₆)-Alkoxy und im Falle cyclischer Reste auch (C₁-C₄)-Alkyl und (C₁-C₄)-Haloalkyl substituiert sind, bedeuten.

In den nutzpflanzenschützenden Mitteln sind solche Verbindungen der allgemeinen Formel l bevorzugt, in der
X CH;
R¹ Wasserstoff, (C₁-C₆)-Alkyl, (C₃-C₆)-Cycloalkyl, (C₂-C₆)-Alkenyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sechs letztgenannten Reste gegebenenfalls durch einen oder mehrere, gleiche oder verschiedene Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-AIkyl und (C₁-C₄)-Haloalkyl substituiert sind;
R² Wasserstoff, (C₁-C₆)-Alkyl, (C₂-C₆)-Alkenyl, (C₂-C₆)-Alkinyl, wobei die drei letztgenannten Reste gegebenenfalls durch einen oder mehrere, gleiche oder verschiedene Substituenten aus der Gruppe Halogen, Hydroxy, (C₁-C₄)-Alkyl, (C₁-C₄)-Alkoxy und (C₁-C₄)-Alkylthio substituiert sind;
R³ Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl;
R⁴ Wasserstoff;
R⁵ Halogen, Nitro, (C₁-C₄)-Alkyl, (C₁-C₄)-Haloalkyl, (C₁-C₄)-Haloalkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl;
n 0, 1 oder 2 und
m 1 oder 2
bedeuten.

Besonders bevorzugt sind in den nutzpflanzenschützenden Mitteln solche Verbindungen der allgemeinen Formel l, in der
R¹ Wasserstoff, (C₁-C₆)-Alkyl, (C₃-C₆)-Cycloalkyl, wobei die zwei letztgenannten Reste gegebenenfalls durch einen oder mehrere, gleiche oder verschiedene Substituenten aus der Gruppe Halogen, (C₁-C₄)-Alkoxy, (C₁-C₆)-Haloalkoxy und (C₁-C₄)-Alkylthio und im Falle cyclischer Reste auch (C₁-C₄)-Alkyl und (C₁-C₄)-Haloalkyl substituiert sind,
bedeutet.

In den nutzpflanzenschützenden Mitteln sind solche Verbindungen der allgemeinen Formel l ganz besonders bevorzugt, in der sich die Sulfamoylgruppe in 4-Position des Phenylringes befindet.
Die erfindungsgemäßen Mittel können mindestens ein Pestizid enthalten. Als Pestizide kommen beispielsweise Herbizide, Insektizide, Fungizide, Akarizide und Nematizide, welche jeweils bei alleiniger Anwendung phytotoxische Schäden an den Kulturpflanzen ergeben würden oder bei denen eine Schädigung wahrscheinlich wäre, in Frage. Von besonderem Interesse sind entsprechende pestizide Wirkstoffe aus den Gruppen der Herbizide und Insektizide, insbesondere Herbizide.

Bevorzugt sind nutzpflanzenschützende Mittel enthaltend mindestens ein Herbizid und mindestens eine Verbindung der allgemeinen Formel l.

Für den Fall, daß die erfindungsgemäßen Mittel Pestizide enthalten, werden diese Mittel nach entsprechender Verdünnung entweder direkt auf die Anbaufläche, auf die bereits gekeimten Schad- und/oder Nutzpflanzen oder auf die bereits aufgelaufenen Schad- und/oder Nutzpflanzen appliziert. Für den Fall, daß die erfindungsgemäßen Mittel kein Pestizid enthalten, können diese Mittel
- im sogenannten Tankmix-Verfahren - d.h. unmittelbar vor dem Aufbringen auf die zu behandelnde Fläche erfolgt beim Anwender die Vermischung und Verdünnung der separat käuflichen Produkte nutzpflanzenschützendes Mittel und Pestizid -, oder
- zeitlich vor der Anwendung eines Pestizids, oder
- zeitlich nach der Anwendung eines Pestizids, oder
- zur Saatgut-Vorbehandlung, d.h. zur Beizung des Nutzpflanzensaatguts verwendet werden.

Bevorzugt ist die gemeinsame Anwendung von Safener und Pestizid, insbesondere die von Safener und Herbizid als Fertigformulierung oder die Anwendung im Tankmix-Verfahren.

Die erfindungsgemäßen Verbindungen der Formel l können zur gemeinsamen Anwendung mit Pestiziden gleichzeitig oder in beliebiger Reihenfolge mit den Wirkstoffen ausgebracht werden und sind dann in der Lage, schädliche Nebenwirkungen dieser Wirkstoffe bei Kulturpflanzen zu reduzieren oder völlig aufzuheben, ohne die Wirksamkeit dieser Wirkstoffe gegen unerwünschte Schadorganismen zu beeinträchtigen. Dabei können auch Schädigungen, welche durch die Anwendung mehrerer Pestizide entstehen, z.B. durch mehrere Herbizide oder durch Herbizide in Kombination mit Insektiziden oder Fungiziden, wesentlich reduziert oder völlig aufgehoben werden. Hierdurch kann das Einsatzgebiet herkömmlicher Pestizide ganz erheblich erweitert werden.

Insektizide, die allein oder gemeinsam mit Herbiziden Pflanzenschädigungen verursachen können, sind beispielsweise folgende:
Organophosphate z.B. Terbufos (Counter® ), Fonofos (Dyfonate® ), Phorate (Thimet® ), Chlorpyriphos (Reldan® ), Carbamate, wie Carbofuran (Furadan® ), Pyrethroid-Insektizide, wie Tefluthrin (Force® ), Deltamethrin (Decis® ) und Tralomethrin (Scout® ) sowie andere insektizide Mittel mit andersartigem Wirkmechanismus.

Herbizide, deren phytotoxische Nebenwirkungen auf Kulturpflanzen mittels Verbindungen der Formel l herabgesetzt werden können, sind z.B. Herbizide aus der Gruppe der Carbamate, Thiocarbamate, Halogenacetanilide, substituierte Phenoxy-, Naphthoxy- und Phenoxyphenoxycarbonsäure-Derivate sowie Heteroaryloxy-phenoxyalkancarbonsäure-Derivate, wie Chinolyloxy-, Chinoxalyloxy-, Pyridyloxy-, Benzoxazolyloxy- und Benzthiazolyloxyphenoxyalkancarbonsäureester, Cyclo-hexandionabkömmlinge, Imidazolinone, Pyrimidinyloxypyridincarbonsäure-Derivate, Pyrimidyloxy-benzoesäure-Derivate, Sulfonylharnstoffe, Triazolo-pyrimidin-sulfonamid-Derivate sowie S-(N-Aryl-N-alkylcarbamoylmethyl)-dithiophosphorsäureester. Bevorzugt sind dabei Phenoxyphenoxy- und Heteroaryloxyphenoxycarbonsäureester und -salze, Sulfonylharnstoffe, Imidazolinone sowie Herbizide, die gemeinsam mit ALS-Hemmstoffen (Acetolactat-Synthetase-Hemmstoffe) zur Erweiterung des Wirkungsspektrums eingesetzt werden, z.B. Bentazon, Cyanazin, Atrazin, Bromoxynil, Dicamba und andere Blattherbizide.

Geeignete Herbizide, die mit den erfindungsgemäßen Safenern kombiniert werden können, sind beispielsweise:
A) Herbizide vom Typ der Phenoxyphenoxy- und Heteroaryloxyphenoxycarbonsäure-Derivate, wie
   A1) Phenoxyphenoxy- und Benzyloxyphenoxy-carbonsäure-Derivate, z.B. 2-(4-(2,4-Dichlorphenoxy)-phenoxy)-propionsäuremethylester (Diclofop-methyl),
      2-(4-(4-Brom-2-chlorphenoxy)phenoxy)propionsäuremethylester (DE-A 26 01 548),
      2-(4-(4-Brom-2-fluorphenoxy)phenoxy)propionsäuremethylester (US-A 4,808,750),
      2-(4-(2-Chlor-4-trifluormethylphenoxy)phenoxy)propionsäuremethylester (DE-A 24 33 067),
      2-(4-(2-Fluor-4-trifluormethylphenoxy)phenoxy)propionsäuremethylester (US-A 4,808,750),
      2-(4-(2,4-Dichlorbenzyl)phenoxy)propionsäuremethylester (DE-A 24 17 487), 4-(4-(4-Trifluormethylphenoxy)phenoxy)pent-2-en-säureethylester, 2-(4-(4-Trifluormethylphenoxy)phenoxy)propionsäuremethylester (DE-A 24 33 067);
   A2) "Einkernige" Heteroaryloxyphenoxy-alkancarbonsäure-Derivate, z.B.
      2-(4-(3,5-Dichlorpyridyl-2-oxy)phenoxy)propionsäureethylester (EP-A 0 002 925), 2-(4-(3,5-Dichlorpyridyl-2-oxy)phenoxy)propionsäurepropargylester (EP-A 0 003 114),
      2-(4-(3-Chlor-5-trifluormethyl-2-pyridyloxy)phenoxy)propionsäure-methylester (EP-A 0 003 890),
      2-(4-(3-Chlor-5-trifluormethyl-2-pyridyloxy)phenoxy)propionsäureethylester (EP-A 0 003 890),
      2-(4-(5-Chlor-3-fluor-2-pyridyloxy)phenoxy)propionsäurepropargylester (EP-A 0 191 736),
      2-(4-(5-Trifluormethyl-2-pyridyloxy)phenoxy)propionsäurebutylester (Fluazifop-butyl);
   A3) "Zweikernige" Heteroaryloxyphenoxy-alkancarbonsäure-Derivate, z.B.
      2-(4-(6-Chlor-2-chinoxalyloxy)phenoxy)propionsäuremethylester und -ethylester (Quizalofopmethyl und Quizalofopethyl),
      2-(4-(6-Fluor-2-chinoxalyloxy)phenoxy)propionsäuremethylester (s. J. Pest. Sci. Vol. 10, 61 (1985)),
      2-(4-(6-Crlor-2-chinoxalyloxy)phenoxy)propionsäure-2-isopropylidenaminooxyethylester (Propaquizafop),
      2-(4-(6-Chlorbenzoxazol-2-yl-oxy)phenoxy)propionsäureethylester (Fenoxaprop-ethyl), dessen D(+) Isomer (Fenoxaprop-P-ethyl) und 2-(4-(6-Chlorbenzthiazol-2-yloxy)phenoxy)propionsäureethylester (DE-A 26 40 730),
      2-(4-(6-Chlorchinoxalyloxy)phenoxy)propionsäure-tetrahydro-2-furylmethylester (EP-A 0 323 727);
B) Herbizide aus der Reihe der Sulfonylharnstoffe, wie Pyrimidin- oder Triazinylaminocarbonyl-[benzol-, pyridin-, pyrazol-, thiophen- und (alkylsulfonyl)-alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)-alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Halogenalkoxy, Halogenalkyl, Alkylcarbonyl, Alkoxyalkyl, (Alkansulfonyl)alkylamino. Solche geeignete Sulfonylharnstoffe sind beispielsweise
   B1) Phenyl- und Benzylsulfonylharnstoffe und verwandte Verbindungen, z.B.
      1-(2-Chlorphenylsulfonyl)-3-(4-methoxy-6-methyl-1,3,5-triazin-2-yl)harnstoff (Chlorsulfuron),
      1-(2-Ethoxycarbonylphenylsulfonyl)-3-(4-chlor-6-methoxypyrimidin-2-yl)harnstoff (Chlorimuron-ethyl),
      1-(2-Methoxyphenylsulfonyl)-3-(4-methoxy-6-methyl-1,3,5-triazin-2-yl)harnstoff (Metsulfuron-methyl),
      1-(2-Chlorethoxyphenylsulfonyl)-3-(4-methoxy-6-methyl-1,3,5-triazin-2-yl)harn-stoff (Triasulfuron),
      1-(2-Methoxycarbonylphenylsulfonyl)-3-(4,6-dimethylpyrimidin-2-yl)harnstoff (Sulfumeturon-methyl),
      1-(2-Methoxycarbonylphenylsulfonyl)-3-(4-methoxy-6-methyl-1,3,5-triazin-2-yl)-3-methylharnstoff (Tribenuron-methyl),
      1-(2-Methoxycarbonylbenzylsulfonyl)-3-(4,6-dimethoxypyrimidin-2-yl)harnstoff (Bensulfuron-methyl),
      1-(2-Methoxycarbonylphenylsulfonyl)-3-(4,6-bis-(difluormethoxy)pyrimidin-2-yl)-harnstoff, (Primisulfuron-methyl),
      3-(4-Ethyl-6-methoxy-1,3,5-triazin-2-yl)-1-(2,3-dihydro-1,1-dioxo-2-methylbenzo[b]thiophen-7-sulfonyl)harnstoff (EP-A 0 796 83),
      3-(4-Ethoxy-6-ethyl-1,3,5-triazin-2-yl)-1 -(2,3-dihydro-1,1-dioxo-2-methylbenzo[b]-thiophen-7-sulfonyl)harnstoff (EP-A 0 079 683),
      3-(4-Methoxy-6-methyl-1,3,5-triazin-2-yl)-1-(2-methoxycarbonyl-5-jod-phenylsulfonyl)-harnstoff (WO 92/13845),
      DPX-66037, Triflusulfuron-methyl (s. Brighton Crop Prot. Conf. - Weeds - 1995, S. 853),
      CGA-277476, (s. Brighton Crop Prot. Conf. - Weeds - 1995, S. 79),
      Methyl-2-[3-(4,6-dimethoxypyrimidin-2-yl)ureidosulfonyl]-4-methansulfon-amidomethyl-benzoat (WO 95/10507),
      N,N-Dimethyl-2-[3-(4,6-dimethoxypyrimidin-2-yl)ureidosulfonyl]-4-formylaminobenzamid (WO 95/01344);
   B2) Thienylsulfonylhamstoffe, z.B.
      1-(2-Methoxycarbonylthiophen-3-yl)-3-(4-methoxy-6-methyl-1,3,5-triazin-2-yl)harnstoff (Thifensulfuron-methyl);
   B3) Pyrazolylsulfonylharnstoffe, z.B.
      1-(4-Ethoxycarbonyl-1-methylpyrazol-5-yl-sulfonyl)-3-(4,6-dimethoxypyrimidin-2-yl)harnstoff (Pyrazosulfuron-methyl);
      Methyl-3-chlor-5-(4,6-dimethoxypyrimidin-2-ylcarbamoylsulfamoyl)-1-methyl-pyrazol-4-carboxylat (EP-A 0 282 613);
      5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference 'Weeds' 1991, Vol. 1, S. 45 ff.),
      DPX-A8947, Azimsulfuron, (s. Brighton Crop Prot. Conf. 'Weeds' 1995, S. 65);
   B4) Sulfondiamid-Derivate, z.B.
      3-(4,6-Dimethoxypyrimidin-2-yl)-1-(N-methyl-N-methylsulfonylaminosulfonyl)-harnstoff (Amidosulfuron) und dessen Strukturanaloge (EP-A 0 131 258 und Z. Pfl. Krankh. Pfl. Schutz, Sonderheft XII, 489-497 (1990));
   B5) Pyridylsulfonylharnstoffe, z.B.
      1-(3-N,N-Dimethylaminocarbonylpyridin-2-ylsulfonyl)-3-(4,6-dimethoxypyrimidin-2-yl)harnstoff (Nicosulfuron),
      1-(3-Ethylsulfonylpyridin-2-ylsulfonyl)-3-(-(4,6-dimethoxypyrimidin-2-yl)harnstoff (Rimsulfuron),
      2-[3-(4,6-Dimethoxypyrimidin-2-yl)ureidosulfonyl]-6-trifluormethyl-3-pyridincarbonsäuremethylester, Natriumsalz (DPX-KE 459, Flupyrsulfuron, s. Brighton Crop Prot. Conf. Weeds, 1995, S. 49),
      Pyridylsulfonylharnstoffe, wie sie in DE-A 40 00 503 und DE-A 40 30 577 beschrieben sind, vorzugsweise solche der Formel 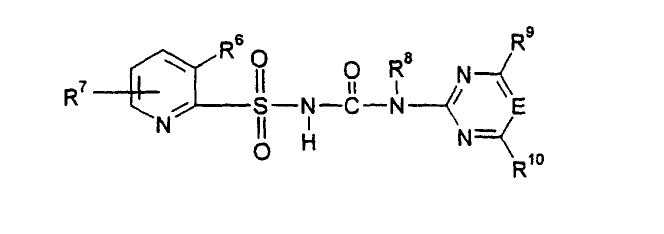 worin
      E CH oder N, vorzugsweise CH,
      R⁶ Jod oder NR¹¹R¹²,
      R⁷ Wasserstoff, Halogen, Cyano, (C₁-C₃)-Alkyl, (C₁-C₃)-Alkoxy, (C₁-C₃)-Halogenalkyl, (C₁-C₃)-Halogenalkoxy, (C₁-C₃)-Alkylthio, (C₁-C₃)-Alkoxy-(C₁-C₃)-alkyl, (C₁-C₃)-Alkoxy-carbonyl, Mono- oder Di-((C₁-C₃)-alkyl)-amino, (C₁-C₃)-Alkylsulfinyl oder -sulfonyl, SO₂-NR^{a}R^{b} oder CO-NR^{a}R^{b}, insbesondere Wasserstoff,
      R^{a}, R^{b} unabhängig voneinander Wasserstoff, (C₁-C₃)-Alkyl, (C₁-C₃)-Alkenyl, (C₁-C₃)-Alkinyl oder zusammen -(CH₂)₄-, -(CH₂)₅- oder -(CH₂)₂-O-(CH₂)₂-,
      R⁸ Wasserstoff oder CH₃,
      R⁹ Halogen, (C₁-C₂)-Alkyl, (C₁-C₂)-Alkoxy, (C₁-C₂)-Halogenalkyl, insbesondere CF₃, (C₁-C₂)-Halogenalkoxy, vorzugsweise OCHF₂ oder OCH₂CF₃,
      R¹⁰ (C₁-C₂)-Alkyl, (C₁-C₂)-Halogenalkoxy, vorzugsweise OCHF₂, oder (C₁-C₂)-Alkoxy, und
      R¹¹ (C₁-C₄)-Alkyl und
      R¹² (C₁-C₄)-Alkylsulfonyl oder
      R¹¹ und R¹² gemeinsam eine Kette der Formel -(CH₂)₃SO₂- oder -(CH₂)₄SO₂-bedeuten, z.B. 3-(4,6-Dimethoxypyrimiden-2-yl)-1-(3-N-methylsulfonyl-N-methylaminopyridin-2-yl)-sulfonylharnstoff, oder deren Salze;
   B6) Alkoxyphenoxysulfonylharnstoffe, wie sie in EP-A 0 342 569 beschrieben sind, vorzugsweise solche der Formel 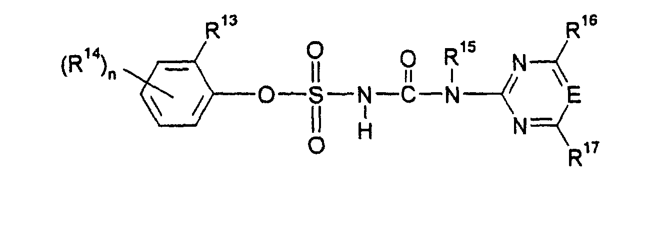 worin
      E CH oder N, vorzugsweise CH,
      R¹³ Ethoxy, Propoxy oder Isopropoxy,
      R¹⁴ Wasserstoff, Halogen, NO₂, CF₃, CN, (C₁-C₄)-Alkyl, (C₁-C₄)-Alkoxy, (C₁-C₄)-Alkylthio oder (C₁-C₃)-Alkoxy)-carbonyl, vorzugsweise in 6-Position am Phenylring,
      n 1, 2 oder 3, vorzugsweise 1,
      R¹⁵ Wasserstoff, (C₁-C₄)-Alkyl oder (C₃-C₄)-Alkenyl,
      R¹⁶, R¹⁷ unabhängig voneinander Halogen, (C₁-C₂)-Alkyl, (C₁-C₂)-Alkoxy, (C₁-C₂)-Halogenalkyl, (C₁-C₂)-Halogenalkoxy oder (C₁-C₂)-Alkoxy-(C₁-C₂)-alkyl, vorzugsweise OCH₃ oder CH₃, bedeuten, z.B. 3-(4,6-Dimethoxypyrimidin-2-yl)-1-(2-ethoxyphenoxy)-sulfonylharnstoff, oder deren Salze;
   B7) Imidazolylsulfonylharnstoffe, z.B.
      MON 37500, Sulfosulfuron (s. Brighton Crop Prot. Conf. 'Weeds', 1995, S: 57), und andere verwandte Sulfonylharnstoff-Derivate und Mischungen daraus;
   C) Chloracetanilide, z.B.
      N-Methoxymethyl-2,6-diethyl-chloracetanilid (Alachlor),
      N-(3-Methoxyprop-2-yl)-2-methyl-6-ethyl-chloracetanilid (Metolachlor),
      N-(3-Methyl-1,2,4-oxadiazol-5-yl-methyl)-chloressigsäure-2,6-dimethylanilid, N-(2,6-Dimethylphenyl)-N-(1-pyrazolylmethyl)-chloressigsäureamid (Metazachlor);
   D) Thiocarbamate, z.B.
      S-Ethyl-N,N-dipropylthiocarbamat (EPTC),
      S-Ethyl-N,N-diisobutylthiocarbamat (Butylate);
   E) Cyclohexandionoxime, z.B.
      3-(1-Allyloxyiminobutyl)-4-hydroxy-6,6-dimethyl-2-oxocyclohex-3-encarbonsäuremethylester, (Alloxydim),
      2-(1-Ethoxyiminobutyl)-5-(2-ethylthiopropyl)-3-hydroxy-cyclohex-2-en-1-on (Sethoxydim),
      2-(1-Ethoxyiminobutyl)-5-(2-phenylthiopropyl)-3-hydroxy-cyclohex-2-en-1-on (Cloproxydim),
      2-(1-(3-Chlorallyloxy)iminobutyl)-5-(2-ethylthiopropyl)-3-hydroxy-cyclohex-2-en-1-on,
      2-(1-(3-Chlorallyloxy)iminopropyl)-5-(2-ethylthiopropyl)-3-hydroxy-cyclohex-2-en-1-on (Clethodim),
      2-(1-Ethoxyiminobutyl)-3-hydroxy-5-(thian-3-yl)-cyclohex-2-enon (Cycloxydim),
      2-(1-Ethoxyiminopropyl)-5-(2,4,6-trimethylphenyl)-3-hydroxy-cyclohex-2-en-1-on (Tralkoxydim);
   F) Imidazolinone, z.B.
      2-(4-lsopropyl-4-methyl-5-oxo-2-imidazolin-2-yl)-5-methylbenzoesäure-methylester und 2-(4-lsopropyl-4-methyl-5-oxo-2-imidazolin-2-yl)-4-methylbenzoesäure (Imazamethabenz),
      5-Ethyl-2-(4-isopropyl-4-methyl-5-oxo-2-imidazolin-2-yl)-pyridin-3-carbonsäure (Imazethapyr),
      2-(4-lsopropyl-4-methyl-5-oxo-2-imidazolin-2-yl)-chinolin-3-carbonsäure (Imazaquin),
      2-(4-lsopropyl-4-methyl-5-oxo-2-imidazolin-2-yl)-pyridin-3-carbonsäure (Imazapyr), 5-Methyl-2-(4-isopropyl-4-methyl-5-oxo-2-imidazolin-2-yl)-pyridin-3-carbonsäure (Imazethamethapyr);
   G) Triazolopyrimidinsulfonamid-Derivate, z.B.
      N-(2,6-Difluorphenyl)-7-methyl-1,2,4-triazolo[1,5-c]pyrimidin-2-sulfonamid (Flumetsulam),
      N-(2,6-Dichlor-3-methylphenyl)-5,7-dimethoxy-1,2,4-triazolo[1,5-c]pyrimidin-2-sulfonamid,
      N-(2,6-Difluorphenyl)-7-fluor-5-methoxy-1,2,4-triazolo[1,5-c]pyrimidin-2-sulfonamid, N-(2,6-Dichlor-3-methylphenyl)-7-chlor-5-methoxy-1,2,4-triazolo[1,5-c]pyrimidin-2-sulfonamid,
      N-(2-Chlor-6-methoxycarbonyl)-5,7-dimethyl-1,2,4-triazolo[1,5-c]pyrimidin-2-sulfonamid (EP-A 0 343 752, US-A 4,988,812);
   H) Benzoylcyclohexandione, z.B.
      2-(2-Chlor-4-methylsulfonylbenzoyl)-cyclohexan-1,3-dion (SC-0051, EP-A 0 137 963), 2-(2-Nitrobenzoyl)-4,4-dimethyl-cyclohexan-1,3-dion (EP-A 0 274 634),
      2-(2-Nitro-3-methylsulfonylbenzoyl)-4,4-dimethylcyclohexan-1,3-dion (WO 91/13548);
   l) Pyrimidinyloxy-pyridincarbonsäure- bzw. Pyrimidinyloxybenzoesäure-Derivate, z.B.
      3-(4,6-Dimethoxypyrimidin-2-yl)-oxy-pyridin-2-carbonsäurebenzyl-ester (EP-A 0 249 707),
      3-(4,6-Dimethoxypyrimidin-2-yl)-oxy-pyridin-2-carbonsäuremethylester (EP-A 0 249 707),
      2,6-Bis[(4,6-dimethoxypyrimidin-2-yl)-oxy]-benzoesäure (EP-A 0 321 846),
      2,6-Bis[(4,6-dimethoxypyrimidin-2-yl)-oxy]-benzoesäure-1-(ethoxycarbonyloxyethyl)-ester (EP-A 0 472 113);
   J) S-(N-Aryl-N-alkyl-carbamoylmethyl)-dithiophosphonsäureester, wie S-[N-(4-Chlorphenyl)-N-isopropyl-carbamoylmethyl]-O,O-dimethyl-dithiophosphat (Anilophos).

Die Herbizide der Gruppen A bis J sind beispielsweise aus den oben jeweils genannten Schriften und aus "The Pesticide Manual", The British Crop Protection Council and the Royal Soc. of Chemistry, 10th Edition, 1994, "Agricultural Chemicals Book II - Herbicides -", by W.T. Thompson, Thompson Publications, Fresno CA, USA 1990 und "Farm Chemicals Handbook '90", Meister Publishing Company, Willoughby OH, USA,1990, bekannt.

Das Gewichtsverhältnis Safener zu Pestizid kann innerhalb weiter Grenzen variiert werden und liegt vorzugsweise im Bereich von 1:10 bis 10:1, insbesondere 1:10 bis 5:1. Das optimale Gewichtsverhältnis Safener zu Pestizid hängt sowohl von den eingesetzten Wirkstoffen Safener und Pestizid als auch von der Art der zu schützenden Nutzpflanzen ab. Die erforderliche Aufwandmenge an Safener kann je nach verwendetem Pestizid und Art der zu schützenden Nutzpflanze innerhalb weiter Grenzen variiert werden und liegt in der Regel im Bereich von 0,001 bis 5 kg, vorzugsweise 0,005 bis 0,5 kg Safener je Hektar. Die für eine erfolgreiche Behandlung notwendigen Mengen und Gewichtsverhältnisse können durch einfache Vorversuche ermittelt werden.

In der Regel können die erfindungsgemäßen Mittel zum Schutz verschiedener Nutzpflanzenkulturen wie Baumwolle, Getreide, Mais, Raps, Reis und Sojabohne eingesetzt werden. Bevorzugte Nutzpflanzenkulturen sind Getreide und Mais.

Ein besonderer Vorteil der erfindungsgemäßen Safener der Formel l ist bei deren Kombination mit Herbiziden aus der Gruppe der Sulfonylharnstoffe und/oder Imidazolinone sowie mit Herbiziden vom Typ der Phenoxyphenoxy- und Heteroaryloxyphenoxy-alkancarbonsäure-Derivate festzustellen. Zahlreiche Herbizide dieser Strukturklassen verursachen nämlich speziell in Getreidekulturen, Mais sowie Reis beträchtliche Schäden an den Nutzpflanzen und können bisher in diesen Kulturen nicht immer eingesetzt werden. Durch die Kombination mit den erfindungsgemäßen Safenern sind auch bei diesen Herbiziden in Getreide, Mais oder Reis hervorragende Selektivitäten zu erreichen.

Die Verbindungen der allgemeinen Formel l und deren Kombinationen mit einem oder mehreren der genannten Pestizide können in Abhängigkeit von den vorgegebenen chemisch-physikalischen und biologischen Parametern auf verschiedene Arten formuliert werden. Als Formulierungsarten sind beispielsweise geeignet:
- Emulgierbare Konzentrate, die durch Auflösen der Wirkstoffe in einem organischen Lösungsmittel, z.B. Butanol, Cyclohexanon, Dimethylformamid, Xylol oder auch höher siedenden Kohlenwasserstoffen oder Mischungen der organischen Lösungsmittel unter Zusatz von einem oder mehreren Tensiden ionischer und/oder nichtionischer Art (Emulgatoren) hergestellt werden. Geeignete Emulgatoren sind beispielsweise alkylarylsulfonsaure Calcium-Salze, Fettsäurepolyglykolester, Alkyarylpolyglykolether, Fettalkoholpolyglykolether, Propylenoxid-Ethylenoxid-Kondensationsprodukte, Alkylpolyether, Sorbitanester und Polyoxyethylensorbitanfettsäureester
- Stäubemittel, die durch Vermahlen der Wirkstoffe mit fein-verteilten festen anorganischen oder organischen Stoffen, z.B. Talkum, natürlichen Tonen, wie Kaolin, Bentonit und Pyrophyllit, Diatomeenerde oder Mehlen erhalten werden
- Auf Wasser oder Öl basierende Suspensionskonzentrate, die beispielsweise durch Naßvermahlung mittels Perlmühlen hergestellt werden können
- Wasserlösliche Pulver
- Wasserlösliche Konzentrate
- Granulate, wie wasserlösliche Granulate, wasserdispergierbare Granulate sowie Granulate für die Streu- und Bodenapplikation
- Spritzpulver, die neben Wirkstoff noch Verdünnungs- oder Inertstoffe und Tenside enthalten
- Kapselsuspensionen und Mikrokapseln
- Ultra-Low-Volume-Formulierungen.

Die oben genannten Formulierungsarten sind dem Fachmann bekannt und werden beispielsweise beschrieben in: K. Martens, "Spray Drying Handbook", 3rd Ed., G. Goodwin Ltd., London. 1979; W. van Valkenburg, "Pesticide Formulations", Marcel Dekker, N.Y. 1973; Winnaker-Küchler, "Chemische Technologie", Band 7, C.

Hauser Verlag München, 4. Auflage 1986; "Perry's Chemical Engineer's Handbook", 5th Ed., McGraw-Hill, N.Y. 1973, Seiten 8-57.

Die notwendigen Formulierungshilfsmittel wie Inertmaterialien, Tenside, Lösungsmittel und weitere Zusatzstoffe sind ebenfalls bekannt und werden beispielsweise beschrieben in: McCutcheon's "Detergents and Emulsifiers Annual", MC Publ. Corp., Ridgewood N.J.; C. Marsden, "Solvents Guide", 2nd Ed., Interscience, N.Y. 1963; H. von Olphen, "Introduction to Clay Colloid Chemistry", 2nd Ed., J. Wiley & Sons, N.Y.; Schönfeldt, "Grenzflächenaktive Äthylenoxidaddukte", Wiss. Verlagsgesellschaft, Stuttgart 1976; Sisley and Wood, "Encyclopedia of Surface Active Agents", Chem. Publ. Co. Inc., N.Y. 1964; Watkins, "Handbook of Insecticide Dust Diluents and Carriers", 2nd Ed., Darland Books, Caldwell N.J.; Winnacker-Küchler, "Chemische Technologie", Band 7, C. Hauser Verlag München, 4. Auflage 1986.

Außer den vorstehend genannten Formulierungshilfsmitteln können die nutzpflanzenschützenden Mittel gegebenenfalls übliche Haft-, Netz-, Dispergier-, Penetrations-, Emulgier-, Konservierungs-, Frostschutz-, Füll-, Träger- und Farbstoffe, Entschäumer, Verdunstungshemmer sowie den pH-Wert oder die Viskosität beeinflussende Mittel enthalten.

Je nach Art der Formulierung enthalten die nutzpflanzenschützenden Mittel in der Regel 0,1 bis 99 Gew.-%, insbesondere 0,2 bis 95 Gew.-%, eines oder mehrerer Safener der allgemeinen Formel l oder eine Kombination von Safener und Pestizid. Weiterhin enthalten sie 1 bis 99,9, insbesondere 4 bis 99,5 Gew.-%, eines oder mehrerer fester oder flüssiger Zusatzstoffe und 0 bis 25, insbesondere 0,1 bis 25 Gew.-% eines Tensids. In emulgierbaren Konzentraten beträgt die Wirkstoffkonzentration, d.h. die Konzentration von Safener und/oder Pestizid, in der Regel 1 bis 90, insbesondere 5 bis 80 Gew.-%. Stäubemittel enthalten üblicherweise 1 bis 30, vorzugsweise 5 bis 20 Gew.-% Wirkstoff. In Spritzpulvern beträgt die Wirkstoffkonzentration in der Regel 10 bis 90 Gew.-%. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u. a. variiert die erforderliche Aufwandmenge der Safener.

Die Verbindungen der allgemeinen Formel l können hergestellt werden, indem man beispielsweise
a) eine Verbindung der allgemeinen Formel II 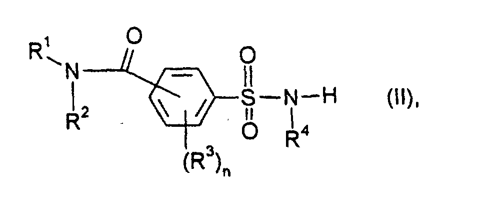 worin R¹, R², R³, R⁴ und n wie oben definiert sind, mit einem Acylierungsmittel, z.B. einem Carbonsäurehalogenid, Carbonsäureanhydrid oder Carbonsäure-imidazolid, der Formel III 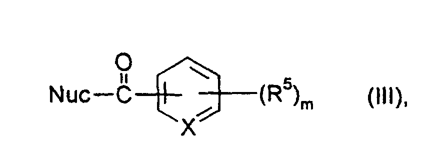 worin Nuc eine Abgangsgruppe und X, R⁵ und m wie oben definiert sind, umsetzt, oder eine Verbindung der allgemeinen Formel lll, in der Nuc für Hydroxy steht, unter Verwendung von Kupplungsreagenzien wie Dicyclohexylcarbodiimid mit dem Sulfonamid der Formel II umsetzt, oder
b) eine Verbindung der allgemeinen Formel IV 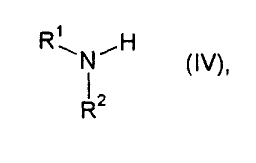
worin R¹ und R² wie oben definiert sind, mit geeigneten aktivierten CarbonsäureDerivaten der allgemeinen Formel V 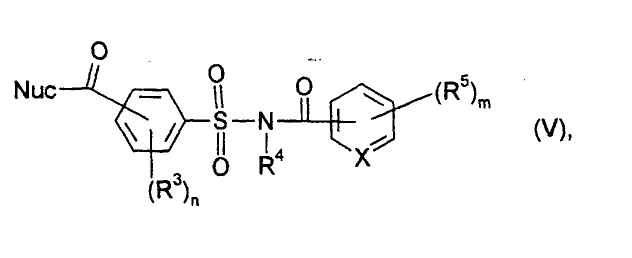 worin X, R³, R⁴, R⁵, n und m wie oben definiert sind und Nuc eine Abgangsgruppe bedeutet, umsetzt.

Die Umsetzungen nach Varianten a) und b) erfolgen vorzugsweise in einem inerten organischen Lösungsmittel in Gegenwart eines säurebindenden Mittels. Als Lösungsmittel eignen sich beispielsweise aprotische polare Lösungsmittel, z.B. Ether, wie THF (Tetrahydrofuran), Dioxan, Acetonitril und Dimethylformamid. Als säurebindende Mittel werden Basen, vorzugsweise organische Basen, wie Triethylamin, Pyridin, Dimethylaminopyridin, DBU (1,8-Diazabicyclo[5.4.0]-undec-7-en), DBN (1,5-Diazabicyclo[4.3.0]non-5-en) und 1,4-Diaza-bicyclo[2.2.2]octan verwendet. Die Reaktionstemperaturen liegen vorzugsweise im Bereich zwischen -20° C und 120° C. Die Verbindungen der allgemeinen Formeln II, III, IV und V sind entweder käuflich oder können nach dem Fachmann bekannten Methoden hergestellt werden.

### BEISPIELE

### 1 FORMUUERUNGSBEISPIELE

### 1.1 STÄUBEMITTEL

Ein Stäubemittel wird erhalten, indem man 10 Gew.-Teile einer Verbindung der Formel l oder eines Wirkstoffgemischs aus einem Herbizid und einem Safener der Formel l und 90 Gew.-Teile Talkum als Inertstoff mischt und in einer Schlagmühle zerkleinert.

### 1.2 WASSERDISPERGIERBARES PULVER

Ein in Wasser leicht dispergierbares, benetzbares Pulver wird erhalten, indem man 25 Gewichtsteile einer Verbindung der Formel l oder eines Wirkstoffgemischs aus einem Herbizid und einem Safener der Formel l, 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 1.3 WASSERDISPERGIERBARES KONZENTRAT

Ein in Wasser leicht dispergierbares Dispersionskonzentrat wird erhalten, indem man 20 Gewichtsteile einer Verbindung der Formel l oder eines Wirkstoffgemischs aus einem Herbizid und einem Safener der Formel l mit 6 Gew.-Teilen Alkylphenolpolyglykolether (® Triton X 207), 3 Gew.-Teilen Isotridecanolpolyglykolether und 71 Gew.-Teilen paraffinischem Mineralöl mischt und in einer Reibkugelmühle auf eine Feinheit von unter 5 Mikron vermahlt.

### 1.4 EMULGIERBARES KONZENTRAT

Ein emulgierbares Konzentrat wird erhalten aus 15 Gew.-Teilen einer Verbindung der Formel l oder eines Wirkstoffgemischs aus einem Herbizid und einem Safener der Formel l, 75 Gew.-Teilen Cyclohexanon als Lösungsmittel und 10 Gew.-Teilen oxethyliertes Nonylphenol als Emulgator.

### 1.5 WASSERDISPERGIERBARES GRANULAT

Ein in Wasserdispergierbares Granulat wird erhalten, indem man

| | |
|---|---|
| 75 | Gewichtsteile eines Safeners der Formel l oder eines Gemischs eines Pestizids und eines Safeners der Formel l, |
| 10 | " ligninsulfonsaures Calcium, |
| 5 | " Natriumlaurylsulfat, |
| 3 | " Polyvinylalkohol und |
| 7 | " Kaolin |

mischt, in einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 | Gewichtsteile eines Safeners der Formel l oder eines Gemischs eines Pestizids und eines Safeners der Formel l, |
| 5 | " 2,2'-dinaphthylmethan-6,6'-disulfonsaures Natrium, |
| 2 | " oleoylmethyltaurinsaures Natrium, |
| 17 | " Calciumcarbonat, |
| 50 | " Wasser und |
| 1 | Gewichtsteil Polyvinylalkohol |

auf einer Kolloidmühle homogenisiert, zerkleinert, dann in einer Perlmühle mahlt und die so erhaltene Suspension in einem Sprühturm mittels einer Einstoffdüse zerstäubt und trocknet.

### 2 HERSTELLUNGSBEISPIELE

### 2.1 4-(2-CHLORBENZOYLSULFAMOYL)-N-PROPYLBENZAMID

### (Beispiel 1-19 aus Tabelle 1)

### 2.1.1 N-PROPYL-4-SULFAMOYLBENZAMID

10 g (0.05 mol) 4-Sulfamoylbenzoesäure werden in 100 ml Tetrahydrofuran bei Raumtemperatur gelöst und mit 8.1 g (0.05 mol) N,N'-Carbonyldiimidazol versetzt. Nach 30 Minuten bei Raumtemperatur und weiteren 30 Minuten bei Rückflußtemperatur werden 2.9 g (0.05 mol) Propylamin bei Raumtemperatur zugegeben. Nach 2 Stunden wird die Reaktionsmischung eingeengt, mit Wasser verrührt, der Niederschlag abgesaugt und getrocknet.
Ausbeute: 8.8 g (70 %); Schmp.: 203° C.

### 2.1.2 4-(2-CHLORBENZOYLSULFAMOYL)-N-PROPYLBENZAMID

1.3 g (8.3 mmol) 2-Chlorbenzoesäure werden in 100 ml Tetrahydrofuran bei Raumtemperatur gelöst und mit 1.3 g (8.3 mmol) N,N'-Carbonyldiimidazol versetzt. Nach 30 Minuten bei Raumtemperatur und weiteren 30 Minuten bei Rückflußtemperatur werden 2.0 g (8.3 mmol) N-Propyl-4-sulfamoylbenzamid bei Rückflußtemperatur zugegeben. Nach 10 Minuten werden 1.2 g (8.3 mmol) 1,5-Diazabicyclo(5.4.0)-5-undecen zugefügt und die Mischung weitere 3 Stunden lang auf Rückflußtemperatur gehalten. Anschließend engt man bis zur Trockne ein, nimmt den Rückstand in 60 ml Acetonitril auf und gibt die Lösung in 60 ml Wasser. Nach Ansäuern der Lösung mit 2N HCI auf einen pH-Wert von 1 wird der Niederschlag abgetrennt, mit Wasser gewaschen und getrocknet.
Ausbeute: 1.8 g (54 %); Schmp.: 210° C.

### 2.2 2,4-DICHLOR-5-(2-CHLORBENZDYLSULFAMOYL)-N-(2-METHOXYETHYL)-BENZAMID

### (Beispiel 2-39 aus Tabelle 2)

### 2.2.1. 2,4-DICHLOR-N-(2-METHOXYETHYL)-5-SULFAMOYLBENZAMID

15 g (56 mmol) 2,4-Dichlor-5-sulfamoylbenzoesäure werden in 350 ml Tetrahydrofuran gelöst und bei Raumtemperatur mit 9 g (56 mmol) N,N'-Carbonyldiimidazol versetzt. Nach 30 Minuten bei Raumtemperatur und weiteren 30 Minuten bei Rückflußtemperatur werden 4.2 g (56 mmol) 2-Methoxyethylamin bei Raumtemperatur zugegeben. Nach 4 Stunden wird die Reaktionsmischung eingeengt, mit Wasser verrührt, der Niederschlag abgesaugt und getrocknet. Ausbeute: 11.7 g (61 %); Schmp.: 158° C.

### 2.2.2. 214-DICHLOR-5-(2-CHLORBENZOYLSULFAMOYL)-N-(2-METHOXYETHYL)-BENZAMID

1.0 g (6.1 mmol) 2-Chlorbenzoesäure werden in 120 ml Tetrahydrofuran bei Raumtemperatur gelöst und mit 1.0 g (6.1 mmol) N,N'-Carbonyldiimidazol versetzt. Nach 30 Minuten bei Raumtemperatur und weiteren 30 Minuten bei Rückflußtemperatur werden 2.0 g (6.1 mmol) 2,4-Dichlor-N-(2-methoxyethyl)-5-sulfamoylbenzamid bei Rückflußtemperatur zugegeben. Nach 10 Minuten werden 0.9 g (6.1 mmol) 1,5-Diazabicyclo(5.4.0)-5-undecen zugefügt und die Mischung weitere 3 Stunden lang auf Rückflußtemperatur gehalten. Anschließend engt man bis zur Trockne ein, nimmt den Rückstand in 60 ml Acetonitril auf und gibt die Lösung in 60 ml Wasser. Nach Ansäuern der Lösung mit 2N HCI auf einen pH-Wert von 1 wird der Niederschlag abgetrennt, mit Wasser gewaschen und getrocknet. Ausbeute: 1.8 g (61%); Schmp.: 181° C.

### 3 BIOLOGISCHE BEISPIELE

### 3.1 BONITIERUNG DER SCHADWIRKUNG

Die Schadwirkung an den Pflanzen wird nach einer Skala von 0-100 % optisch im Vergleich zu Kontrollpflanzen bewertet:
- 0% =: keine erkennbare Wirkung im Vergleich zur unbehandelten Pflanze
- 100% =: behandelte Pflanze stirbt ab.

### 3.2 HERBIZIDWIRKUNG UND SAFENERWIRKUNG IM VORAUFLAUF

Samen bzw. Rhizomstücke von mono- und dikotylen Schadpflanzen sowie von Kulturpflanzen werden in Plastiktöpfen von 9 cm Durchmesser in sandiger Lehmerde ausgelegt und mit Erde abgedeckt. Alternativ hierzu werden im Paddy-Reisanbau vorkommende Schadpflanzen in einem mit Wasser gesättigten Boden kultiviert, wobei so viel Wasser in die Töpfe gefüllt wird, daß das Wasser bis zur Bodenoberfläche oder einige Millimeter darüber steht. Die in Form von Emulsionskonzentraten formulierten erfindungsgemäßen Herbizid-Safener-Wirkstoffkombinationen sowie in parallelen Versuchen die entsprechend formulierten Einzelwirkstoffe werden dann als Emulsionen mit einer Wasseraufwandmenge von umgerechnet 300 l/ha, in unterschiedlichen Dosierungen auf die Oberfläche der Abdeckerde appliziert oder beim Reis ins Bewässerungswasser gegossen. Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 2-3 Wochen im Vergleich zu unbehandelten Kontrollen. Wie die Versuche zeigen, weisen die erfindungsgemäßen herbiziden Mittel, welche z.B. einen Safener der Beispiele 1-17, 1-20, 1-33, 1-67, 1-68, 1-69, 1-70, 1-75, 1-76, 1-84, 1-85, 1-86, 1-117, 1-118, 1-133, 1-150, 1-151, 1-153, 1-162, 1-163, 1-195, 1-197 in Kombination mit dem Sulfonylharnstoffherbizid 3-(4,6-Dimethoxypyrimid-2-yl)-1-[3-(N-methyl-sulfonyl-N-methylamino)-pyridyl-2-yl-sulfonyl]-harnstoff oder 3-(4-Methoxy-6-methyl-1,3,5-triazin-2-yl)-1-(2-methoxycarbonyl-5-jod-phenyl-sulfonyl)-hamstoff (Natriumsalz) oder 1-(2-Methoxycarbonylthiophen-3-yl)-3-(4-methoxy-6-methyl-1,3,5-triazin-2-yl)harnstoff (Thifensulfuron-methyl) oder 1-(3-N,N-Dimethyl-aminocarbonyl-pyridin-2-ylsulfonyl)-3-(4,6-dimethoxypyrimidin-2-yl)harnstoff (Nicosulfuron) oder 1-(3-Ethylsulfonylpyridin-2-ylsulfonyl)-3-(4,6-dimethoxypyrimidin-2-yl)-harnstoff (Rimsulfuron) oder N,N-Dimethyl-2-[3-(4,6-dimethoxypyrimidin-2-yl)-ureidosulfonyl]-4-formylaminobenzamid oder Methyl-2-[3-(4,6-dimethoxy-pyrimidin-2-yl)-ureidosulfonyl]-4-methansulfonamido-methyl-benzoat oder mit dem Imidazolinonherbizid 5-Ethyl-2-(4-isopropyl-4-methyl-5-oxo-2-imidazolin-2-yl)-pyridin-3-carbonsäure (Imazethapyr) oder mit dem Aryloxyphenoxyherbizid 2-(4-(6-Chlorbenzoxazol-2-yl-oxy)-phenoxy)-propionsäureethyl-ester Fenoxaprop-ethyl) oder mit 2,6-Bis[(4,6-dimethoxypyrimidin-2-yl)-oxy]-benzoesäure Natriumsalz im Verhältnis von Herbizid:Safener von 2:1 bis 1:20 enthalten, eine gute herbizide Vorauflaufwirkung gegen ein breites Spektrum von Unkräutern und Ungräsern auf, wobei Schäden an Kulturpflanzen wie Mais, Reis, Weizen oder Gerste oder anderem Getreide im Vergleich zur Anwendung der einzelnen Herbizide ohne Safener wesentlich reduziert sind, d.h. um 30% bis zu 100% weniger Herbizidschäden aufweisen.

### 3.3 HERBIZIDWIRKUNG UND SAFENERWIRKUNG IM NACHAUFLAUF

Samen bzw. Rhizomstücke von mono- und dikotylen Schadpflanzen und von Kulturpflanzen werden in Plastiktöpfen in sandiger Lehmerde ausgelegt, mit Erde abgedeckt und im Gewächshaus unter guten Wachstumsbedingungen angezogen. Alternativ hierzu werden im Paddy-Reisanbau vorkommende Schadpflanzen in Töpfen kultiviert, in denen Wasser bis zu 2 cm über der Bodenoberfläche steht. Drei Wochen nach der Aussaat werden die Versuchspflanzen im Dreiblattstadium behandelt. Die als Emulsionskonzentrate formulierten erfindungsgemäßen Herbizid-Safener-Wirkstoffkombinationen sowie in parallelen Versuchen die entsprechend formulierten Einzelwirkstoffe werden in verschiedenen Dosierungen mit einer Wasseraufwandmenge von umgerechnet 300 l/ha auf die grünen Pflanzenteile gesprüht und nach 2-3 Wochen Standzeit der Versuchspflanzen im Gewächshaus unter optimalen Wachstumsbedingungen die Wirkung der Präparate optisch im Vergleich zu unbehandelten Kontrollen bonitiert. Bei Reis oder bei Schadpflanzen, die im Reisanbau vorkommen, werden die Wirkstoffe auch direkt ins Bewässerungswasser gegeben (Applikation in Analogie zur sogenannten Granulatanwendung) oder auf Pflanzen und ins Bewässerungswasser gesprüht. Wie die Versuche zeigen, weisen die erfindungsgemäßen herbiziden Mittel, welche z.B. einen Safener der Beispiele 1-17, 1-20, 1-33, 1-67, 1-68, 1-69, 1-70, 1-75, 1-76, 1-84, 1-85, 1-86, 1-117, 1-118, 1-133, 1-150, 1-151, 1-153, 1-162, 1-163, 1-195, 1-197 in Kombination mit dem Sulfonylharnstoffherbizid 3-(4,6-Dimethoxypyrimidin-2-yl)-1-[3-(N-methylsulfonyl-N-methylamino)-pyridyl-2-yl-sulfonyl]-harnstoff oder 3-(4-Methoxy-6-methyl-1,3,5-triazin-2-yl)-1-(2-methoxycarbonyl-5-jod-phenylsulfonyl)-harnstoff (Natriumsalz) oder 1-(2-Methoxycarbonylthiophen-3-yl)-3-(4-methoxy-6-methyl-1,3,5-triazin-2-yl)harnstoff (Thifensulfuron-methyl) oder 1-(3-N,N-Dimethylaminocarbonylpyridin-2-ylsulfonyl)-3-(4,6-dimethoxypyrimidin-2-yl)-harnstoff (Nicosulfuron) oder 1-(3-Ethylsulfonylpyridin-2-ylsulfonyl)-3-(4,6-dimethoxypyrimidin-2-yl)harnstoff (Rimsulfuron) oder N,N-Dimethyl-2-[3-(4,6-dimethoxypyrimidin-2-yl)ureidosulfonyl]-4-formylaminobenzamid oder Methyl-2-[3-(4,6-dimethoxypyrimidin-2-yl)ureido-sulfonyl]-4-methansulfonamido-methyl-benzoat oder mit dem Imidazolinonherbizid 5-Ethyl-2-(4-isopropyl-4-methyl-5-oxo-2-imidazolin-2-yl)-pyridin-3-carbonsäure (Imazethapyr) oder mit dem Aryloxyphenoxyherbizid 2-(4-(6-Chlorbenzoxazol-2-yl-oxy)-phenoxy)-propionsäureethylester (Fenoxaprop-ethyl) oder mit 2,6-Bis[(4,6-dimethoxypyrimidin-2-yl)-oxy]-benzoesäure Natriumsalz im Verhältnis von Herbizid:Safener von 2:1 bis 1:20 enthalten, eine gute herbizide Nachauflaufwirkung gegen ein breites Spektrum von Ungräsern und Unkräutern auf, wobei Schäden an Kulturpflanzen wie Mais, Reis, Weizen oder Gerste oder anderem Getreide im Vergleich zur Anwendung der einzelnen Herbizide ohne Safener wesentlich reduziert sind, d.h. um 30% bis zu 100% weniger Herbizidschäden aufweisen.

In den nachfolgenden Tabellen sind beispielhaft eine Reihe von Verbindungen der allgemeinen Formel l aufgeführt, die in analoger Weise zu den obigen Beispielen und den weiter oben erwähnten Methoden erhalten werden können.

In den Tabellen bedeuten:

| | |
|---|---|
| Bu = Butyl | Et = Ethyl |
| Me = Methyl | Nap = Naphthoyl |
| Pr = Propyl | c = cyclo |
| i = iso | s = sekundär |
| t = tertiär | F.P. = Festpunkt |

Ist den Tabellen ein Alkylrest ohne weitere Kennzeichnung aufgeführt, so handelt es sich um den geradkettigen.

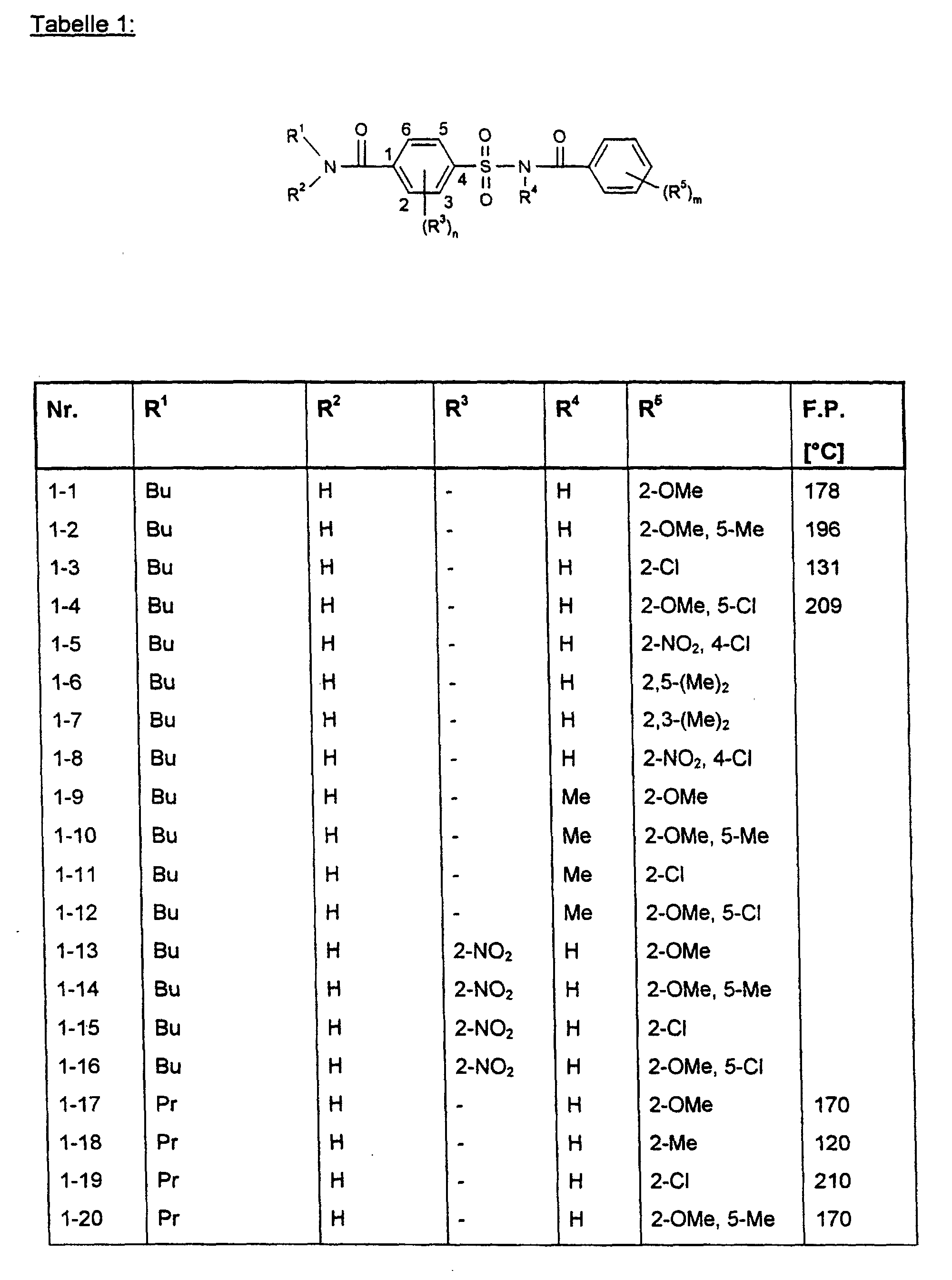

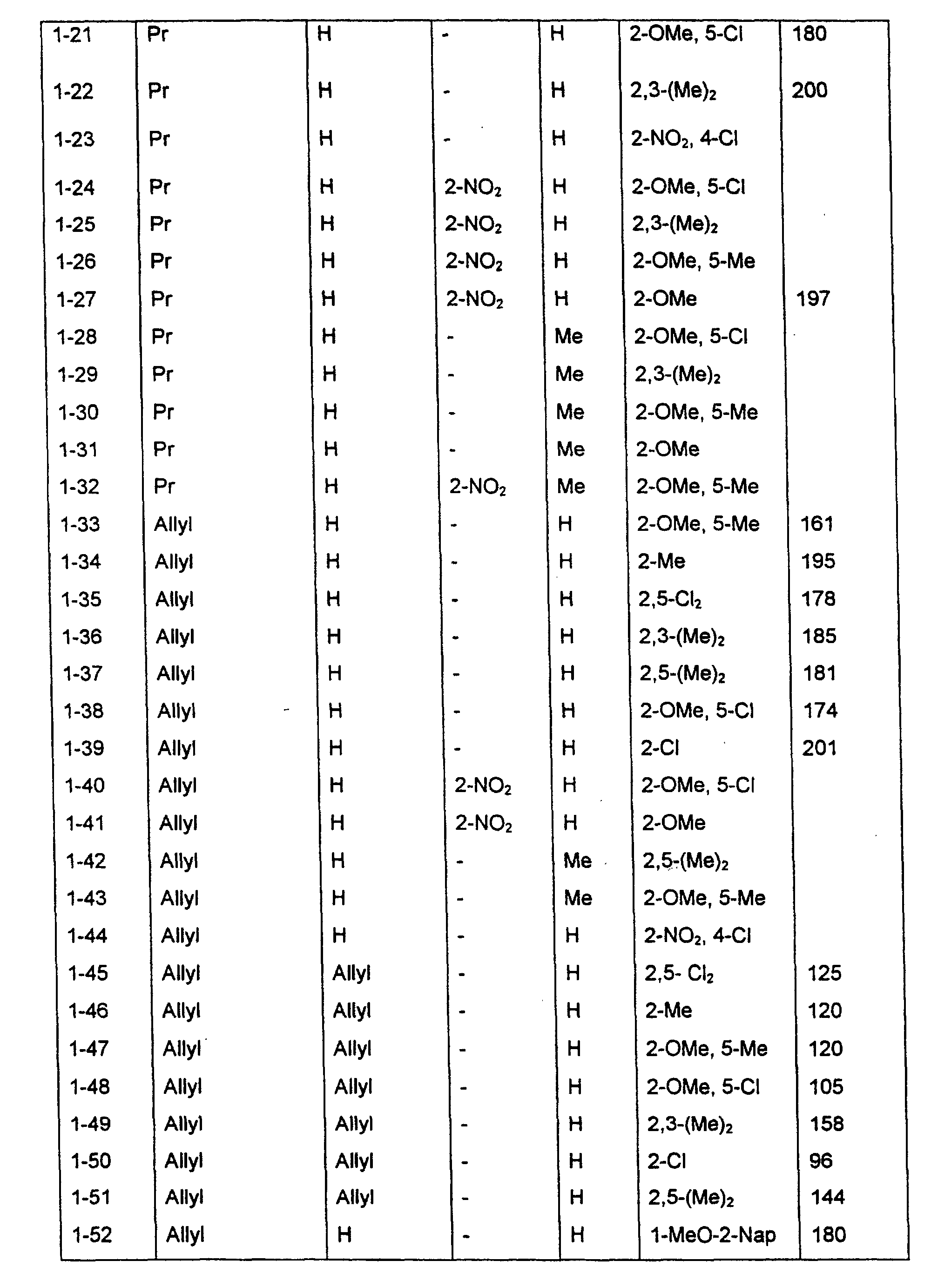

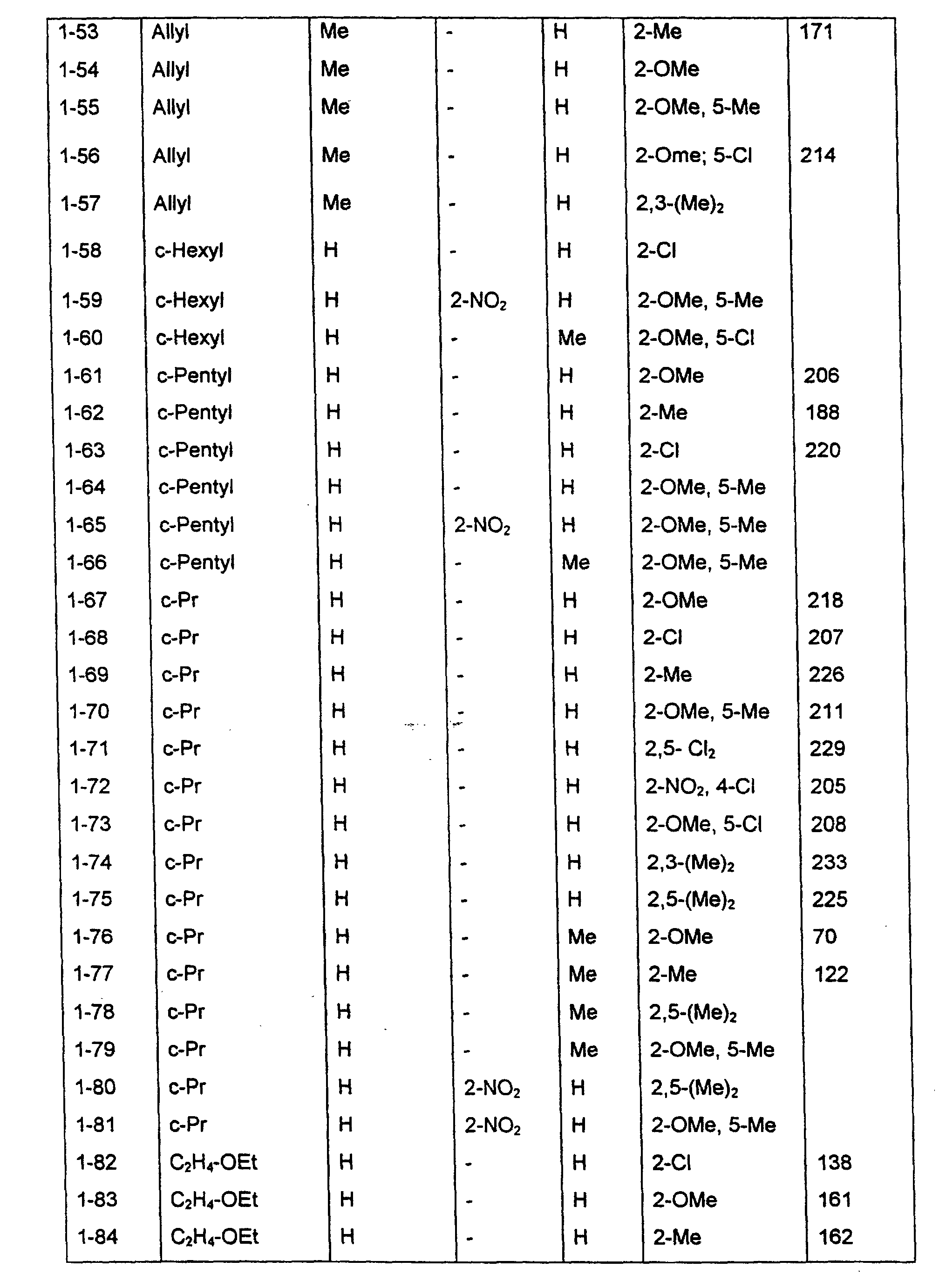

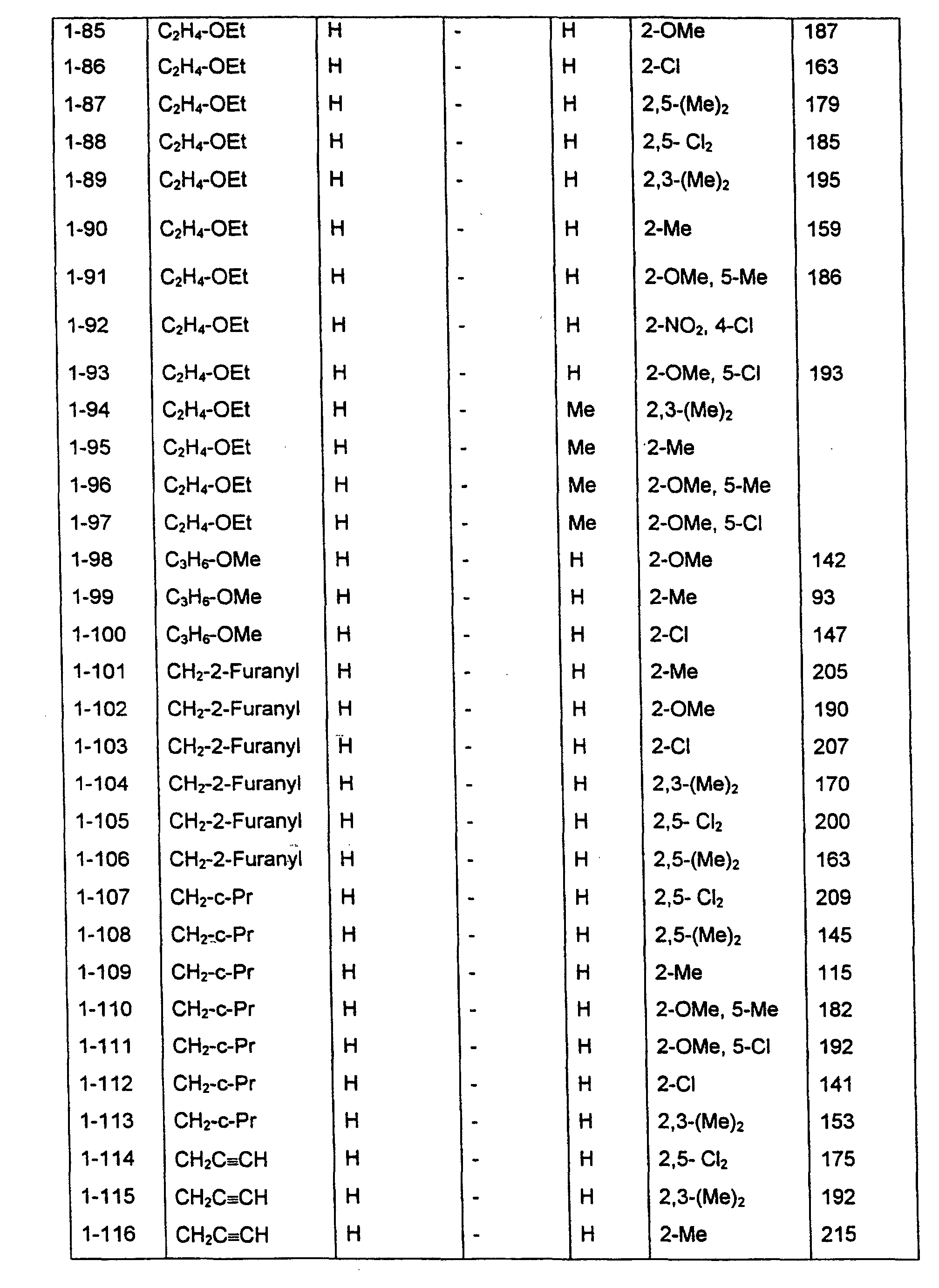

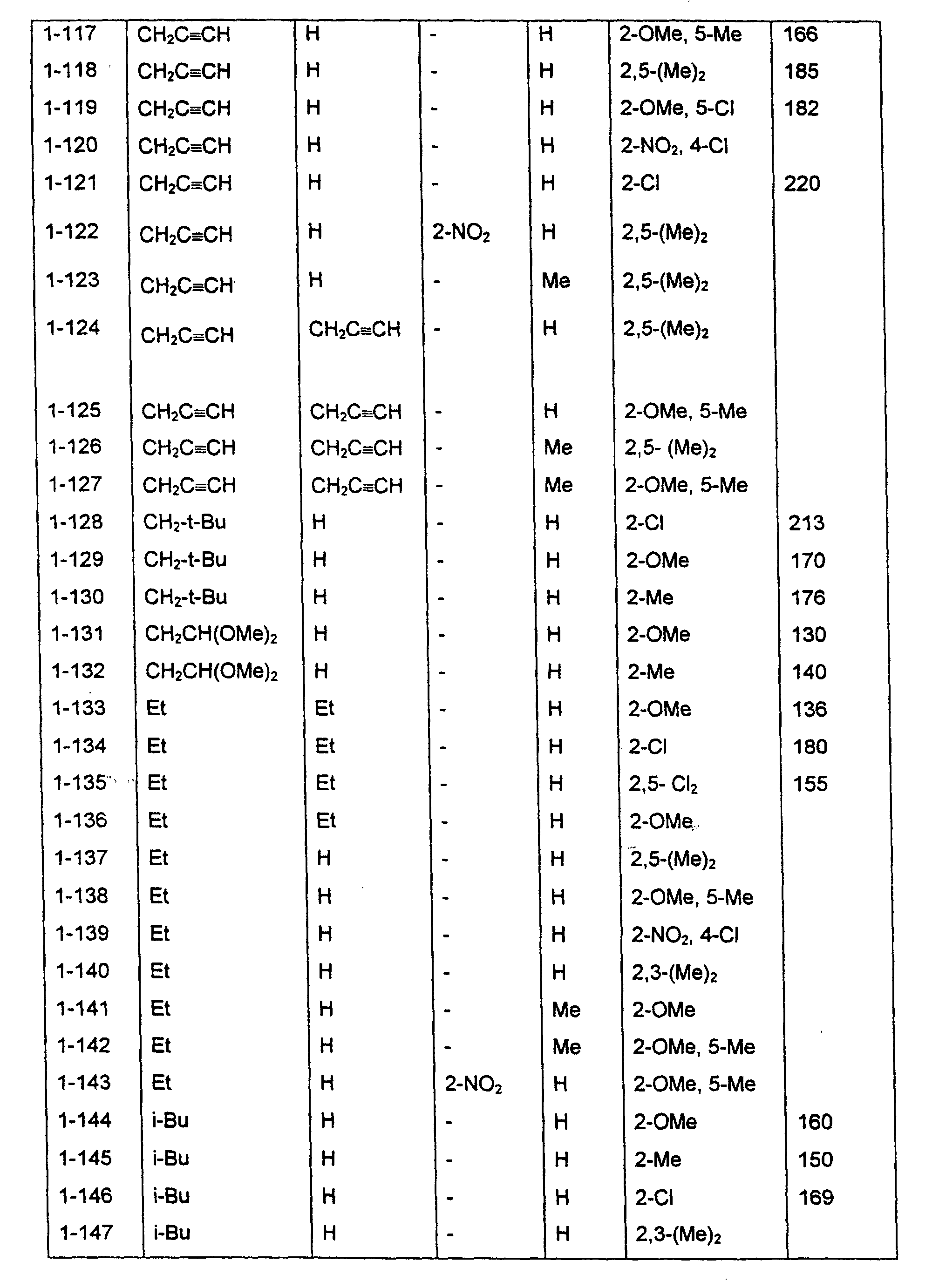

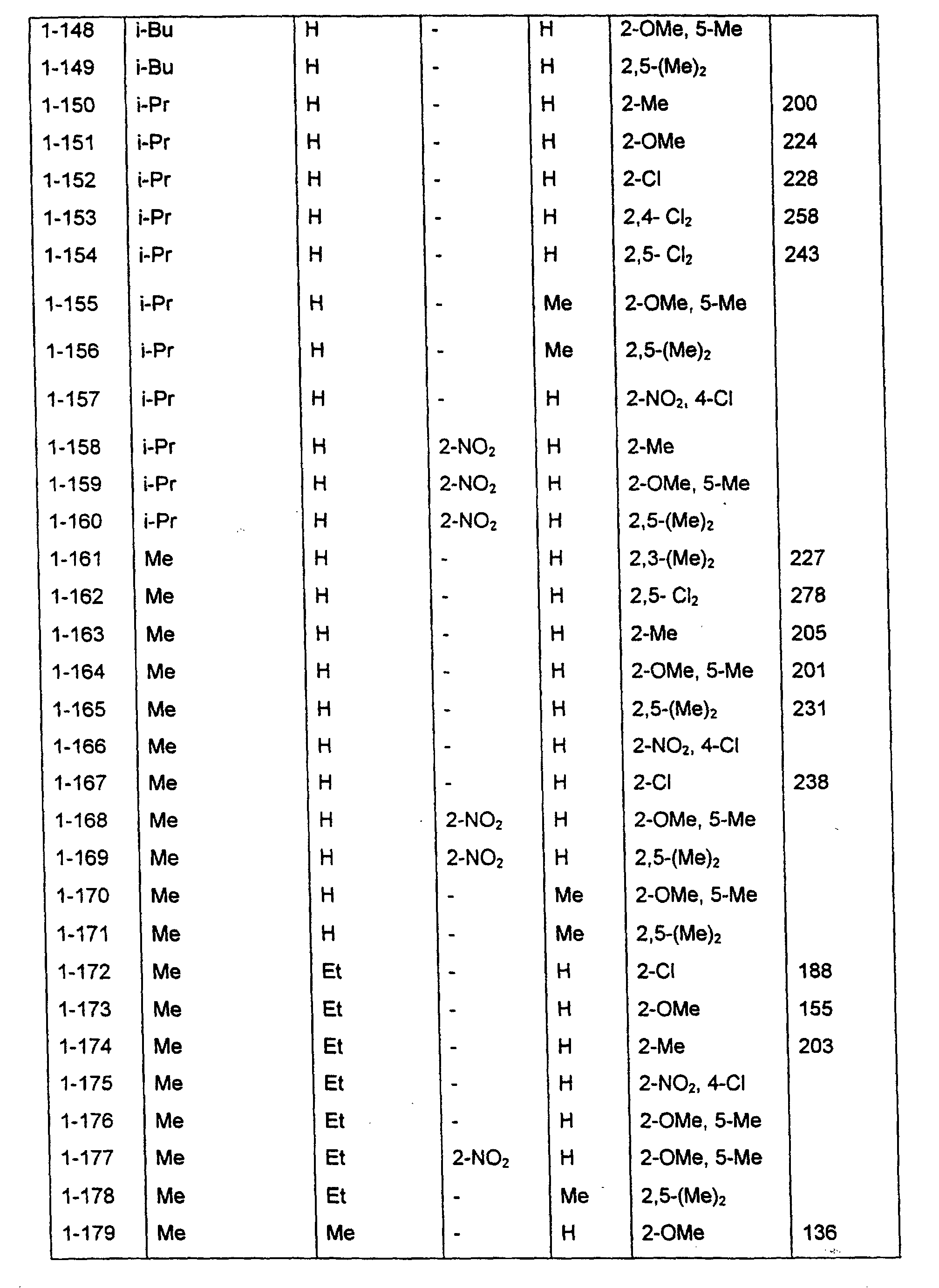

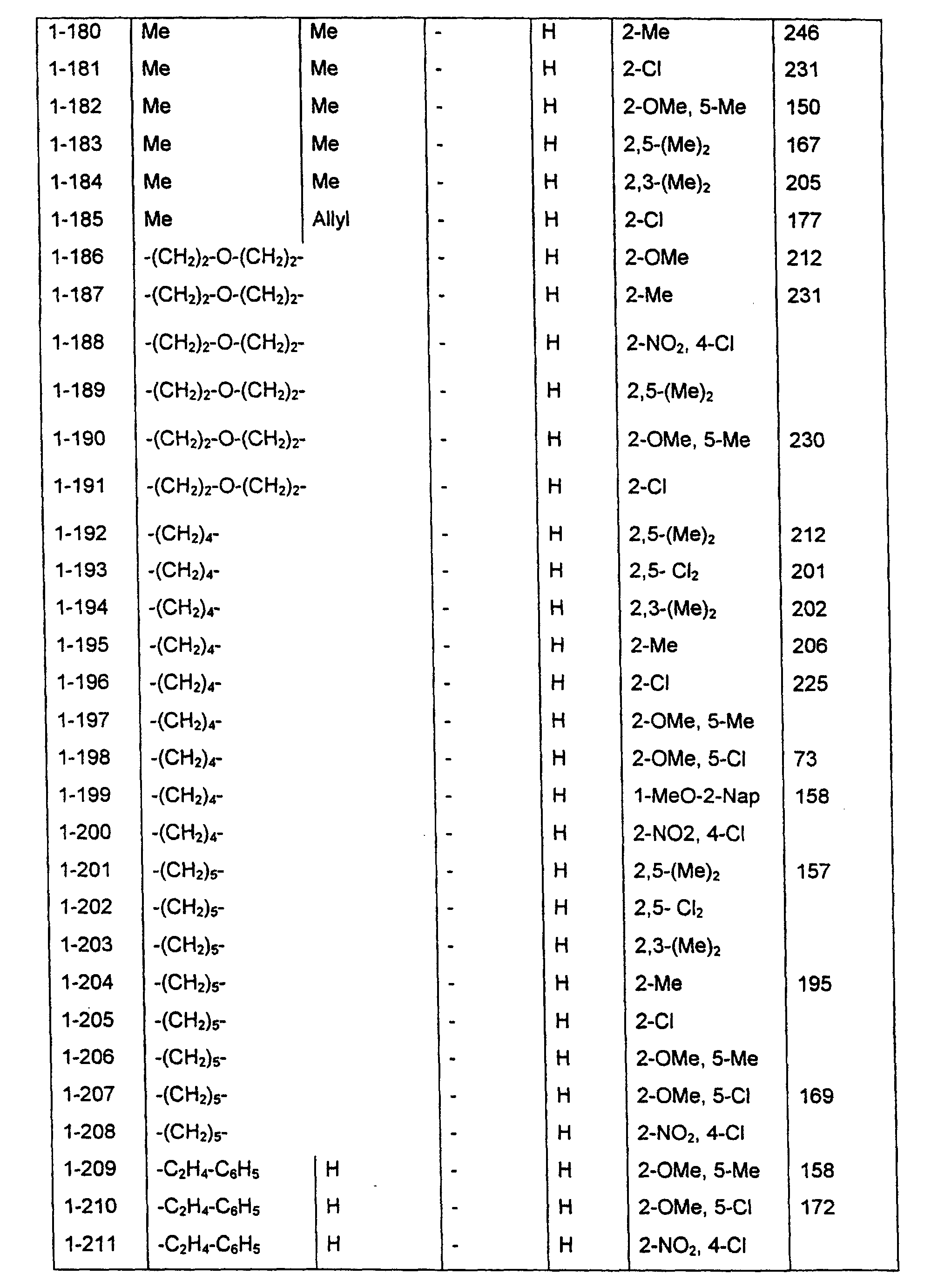

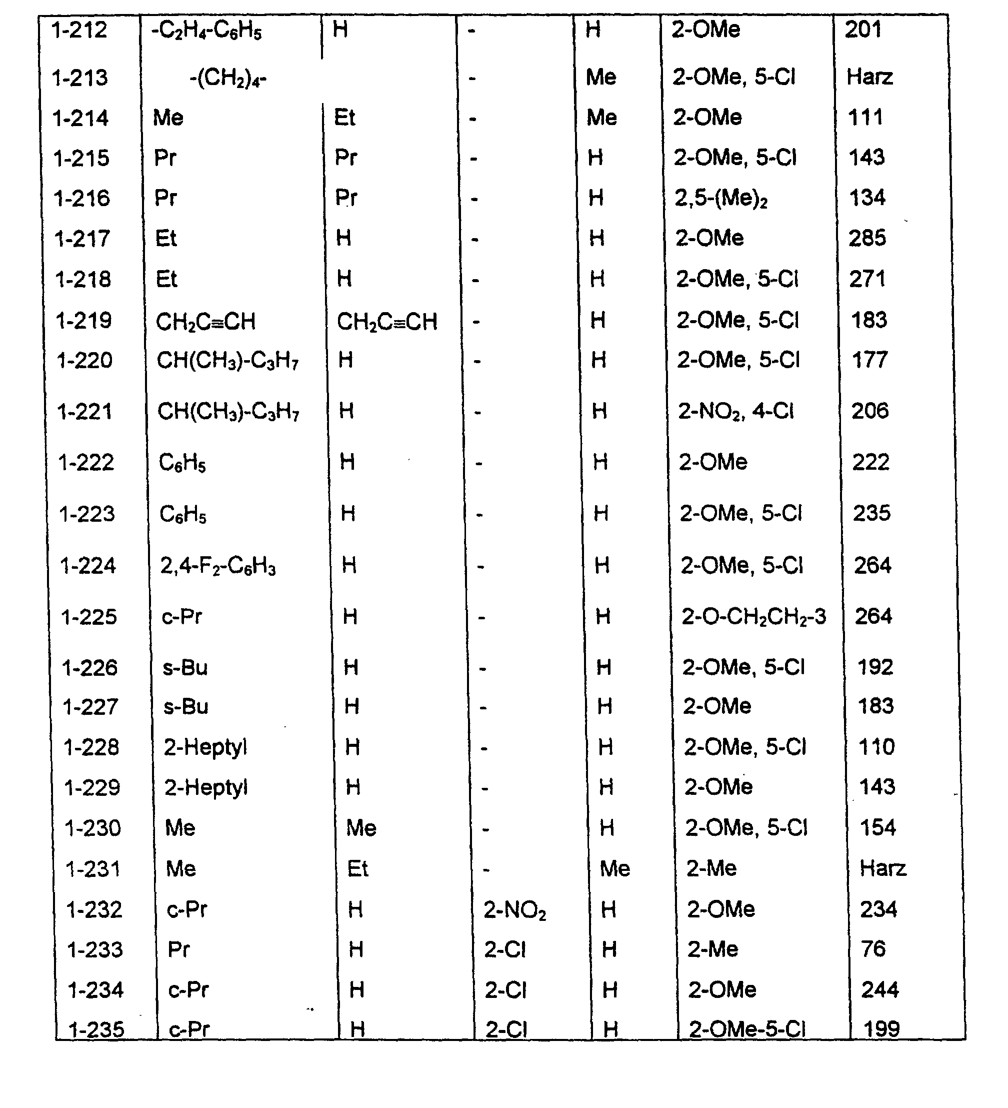

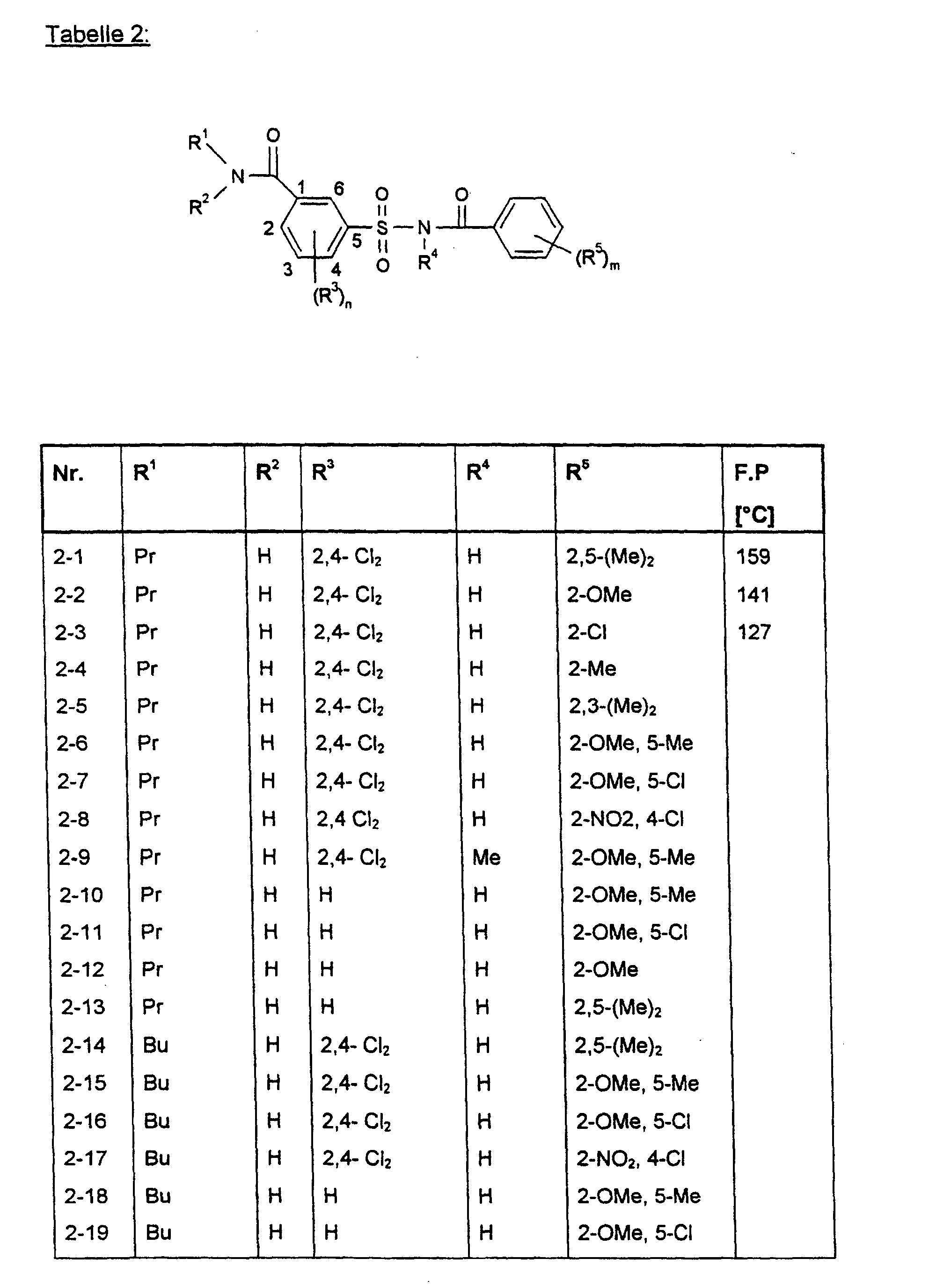

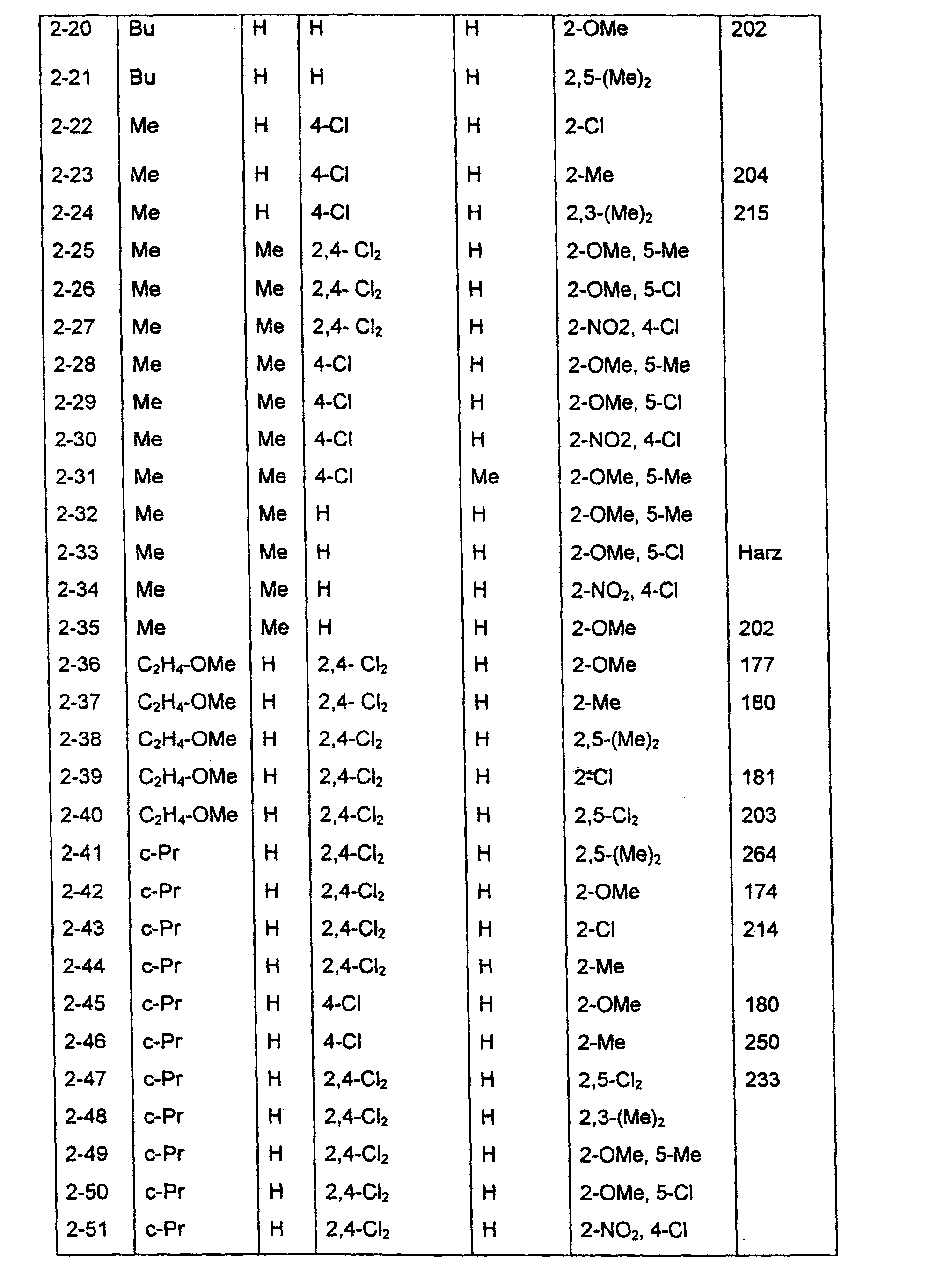

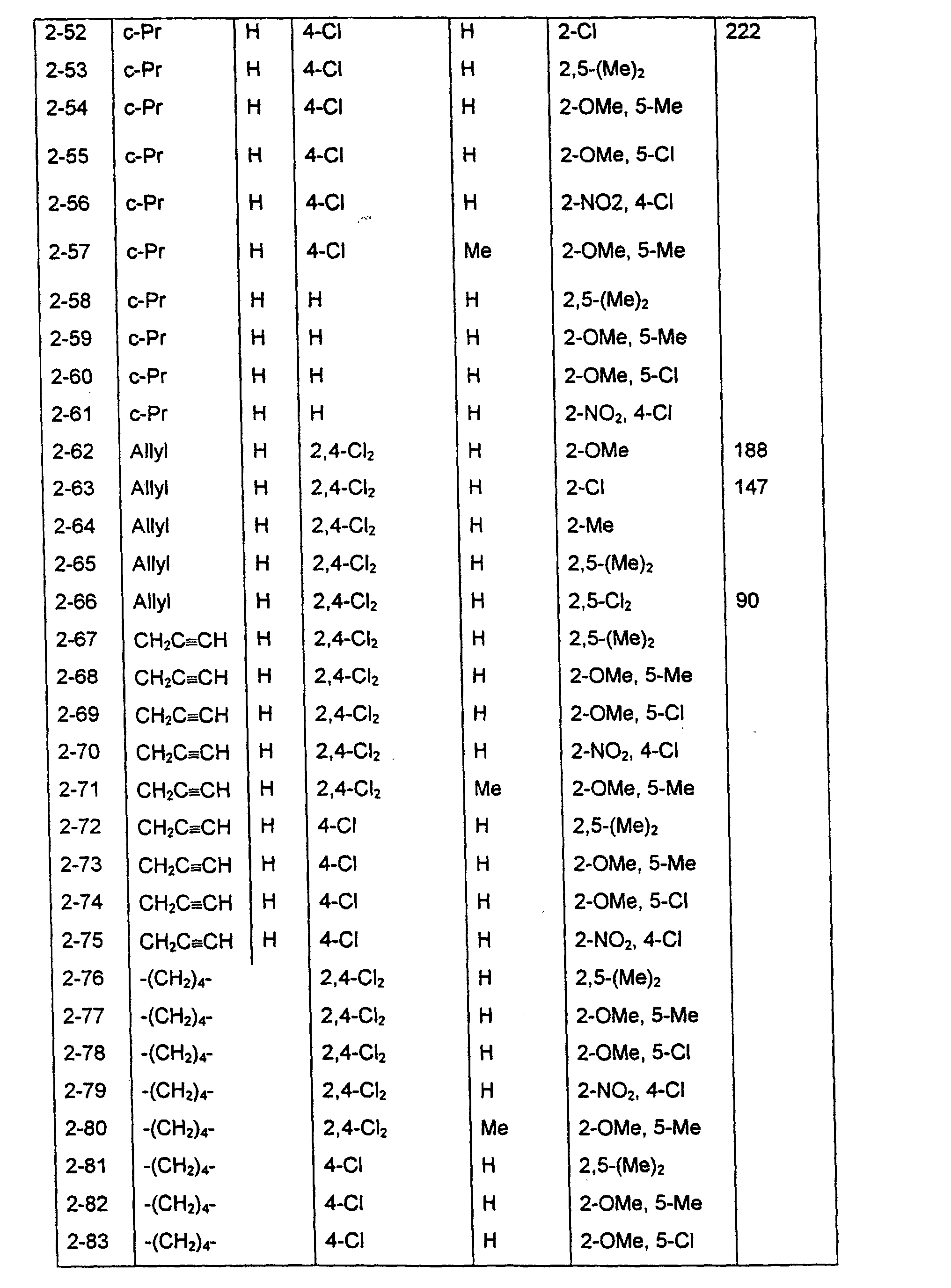

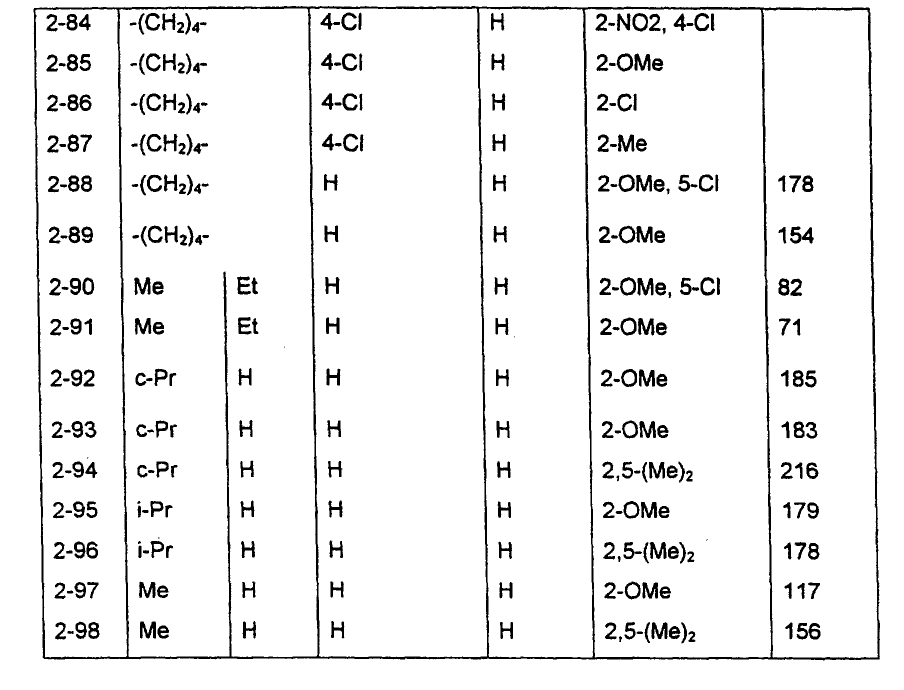

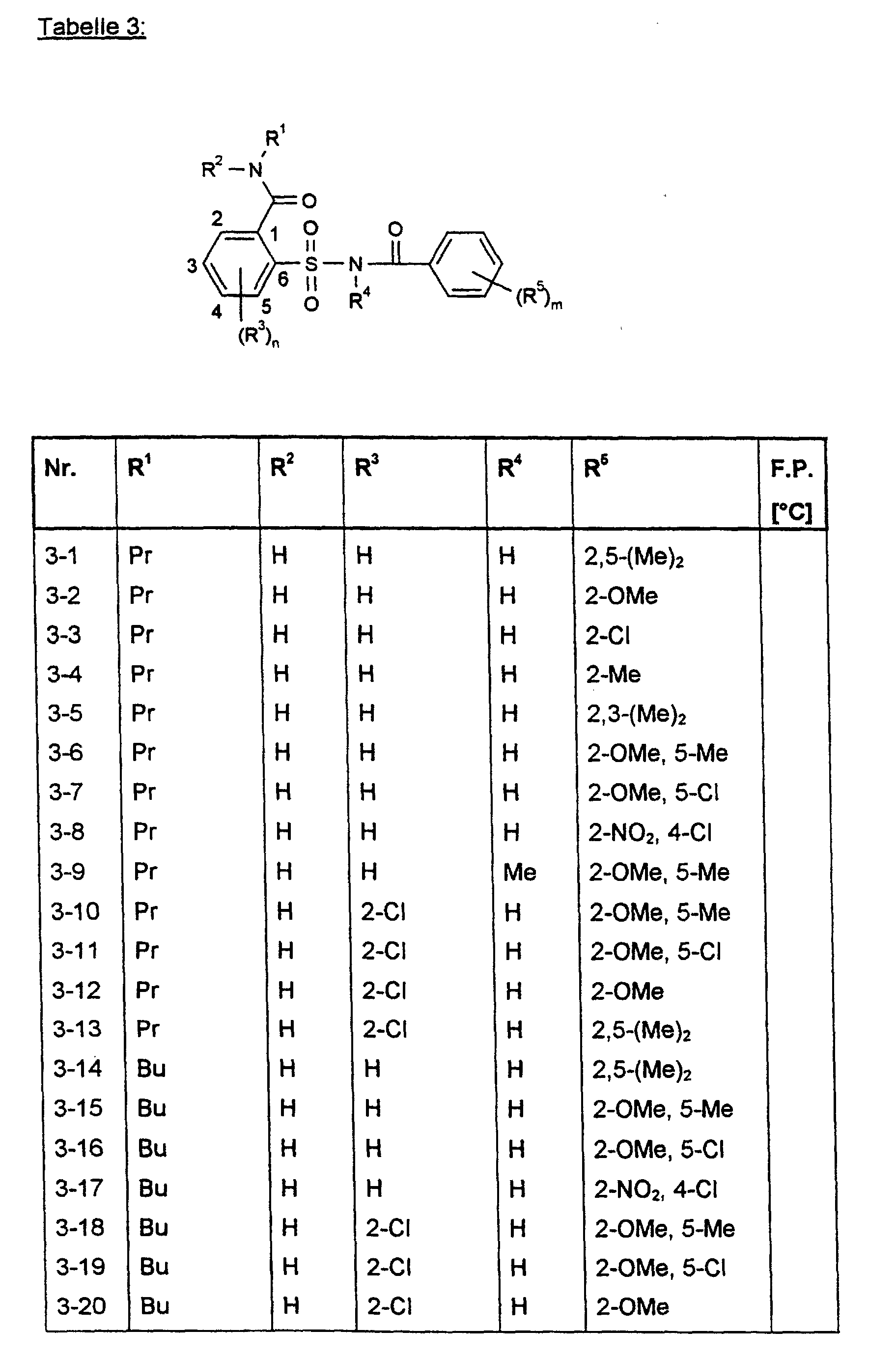

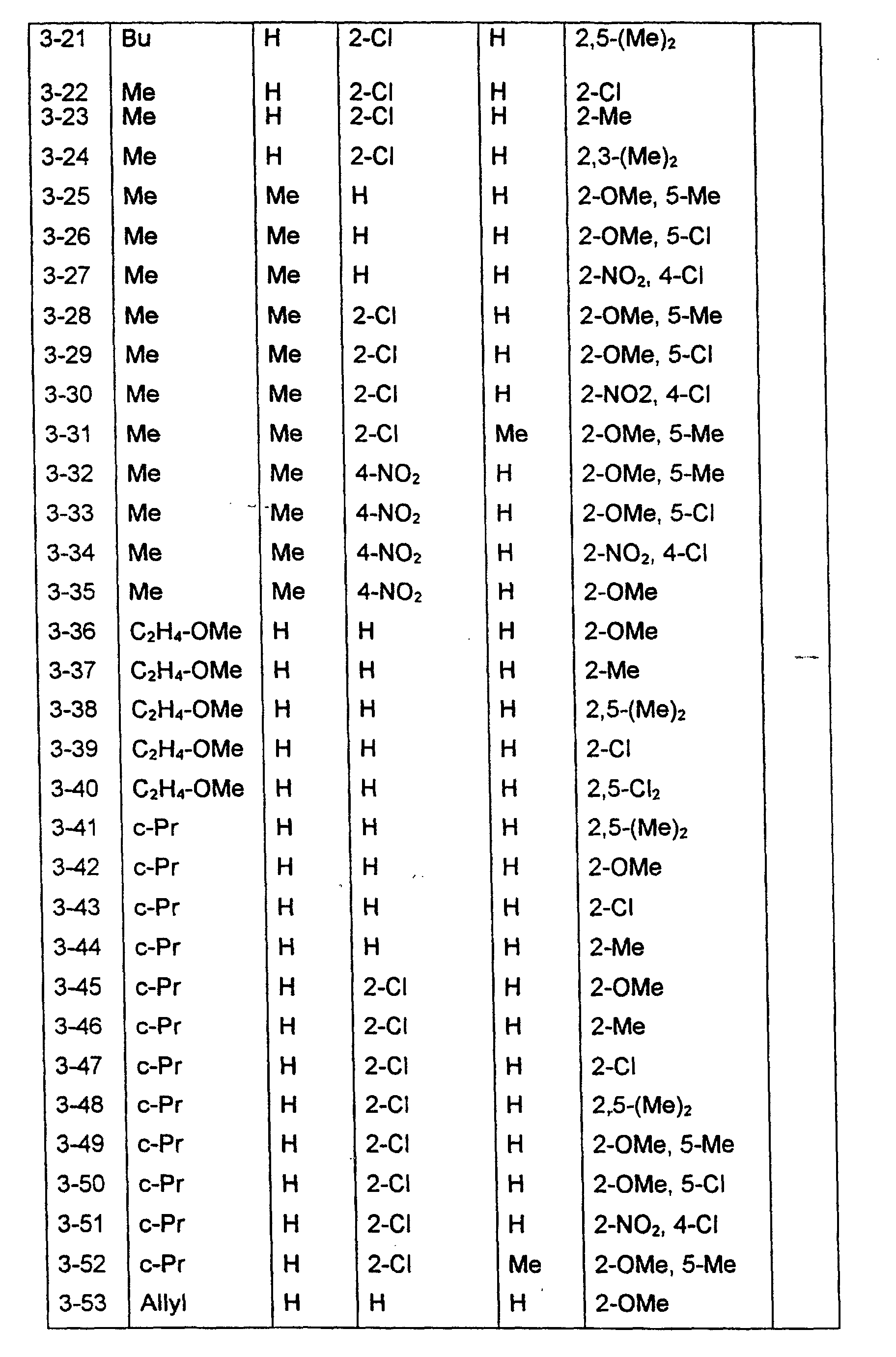

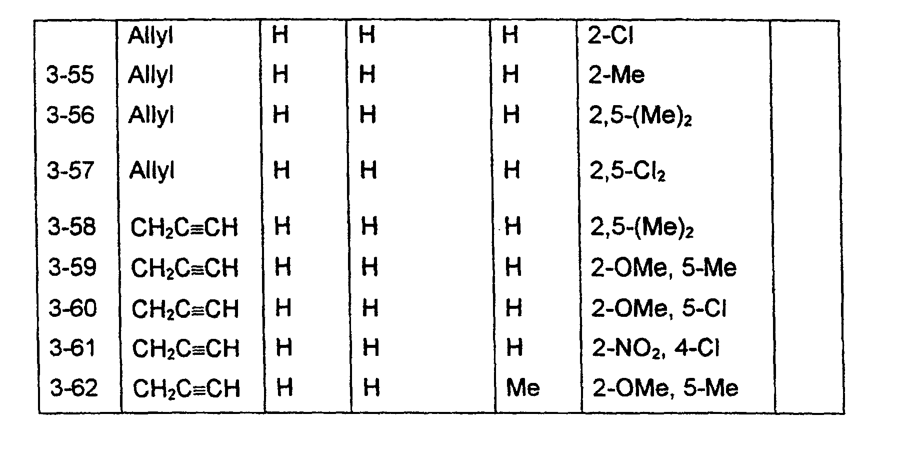

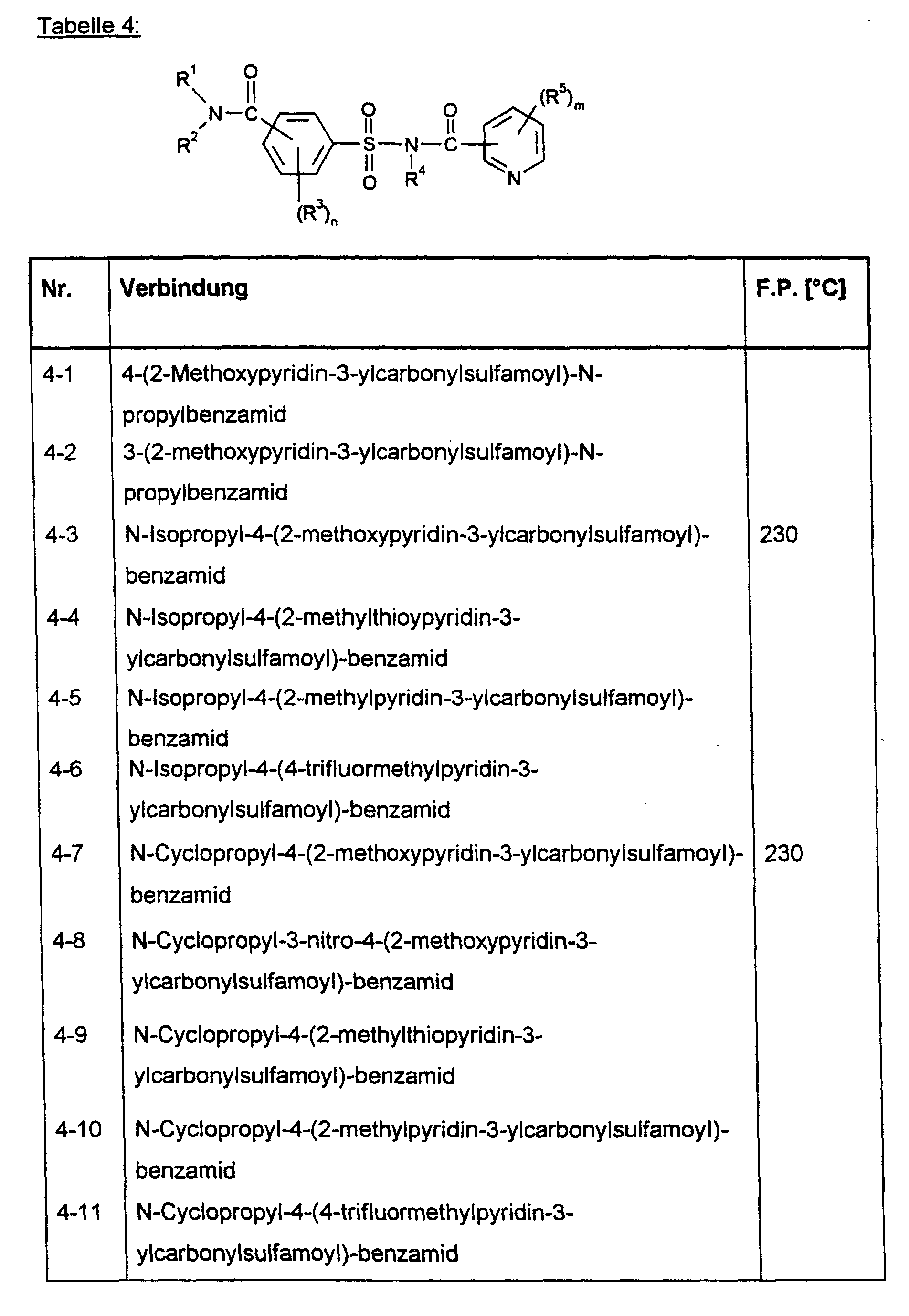

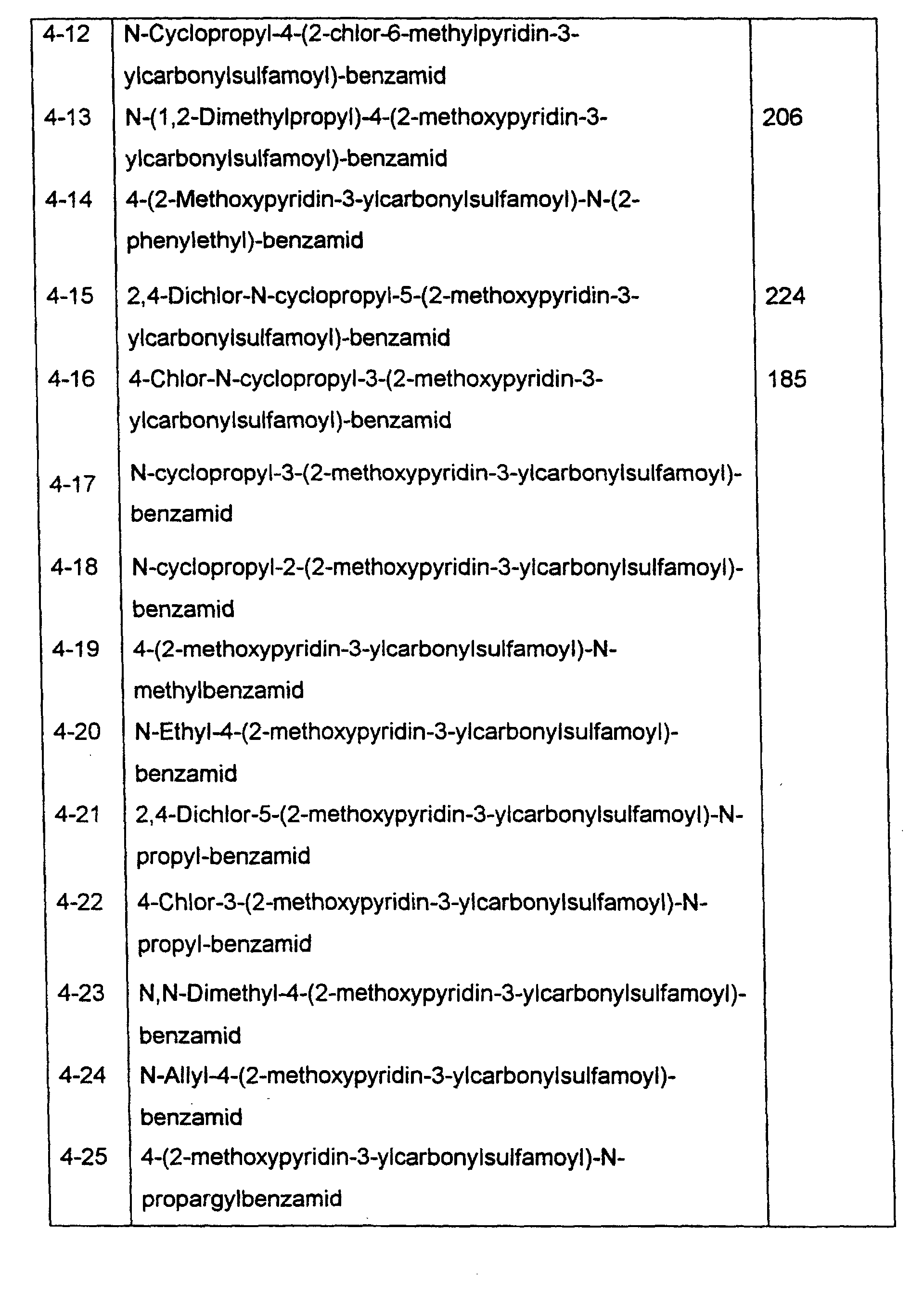

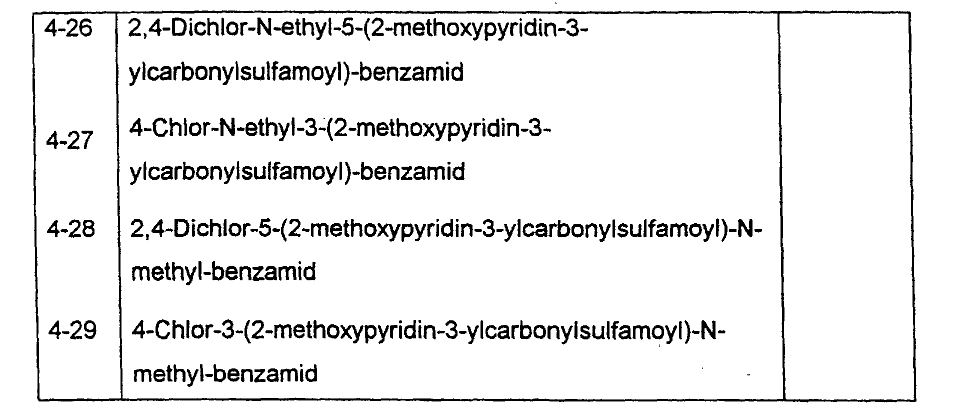

## Patentansprüche

1. Verwendung von Acylsulfamoylbenzoesäureamiden der allgemeinen Formel I, gegebenenfalls auch in seiner Salzform, 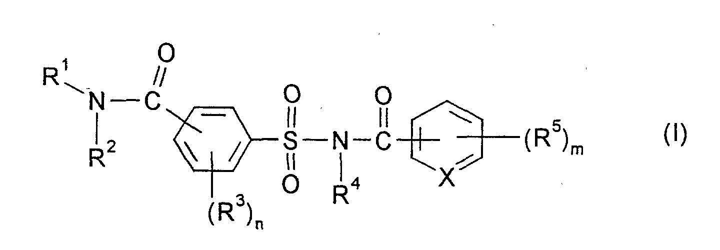 in der
X CH oder N;
R¹ Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
R² Wasserstoff, Hydroxy, (C₁-C₆)-Alkyl, (C₂-C₆)-Alkenyl, (C₂-C₆)-Alkinyl, (C₁-C₆)-Alkoxy, (C₂-C₆)-Alkenyloxy, wobei die fünf letztgenannten Reste gegebenenfalls durch einen oder mehrere, gleiche oder verschiedene Reste aus der Gruppe Halogen, Hydroxy, (C₁-C₄)-Alkyl, (C₁-C₄)-Alkoxy und (C₁-C₄)-Alkylthio substituiert sind, oder
R¹ und R² zusammen mit dem sie tragenden Stickstoffatom einen 3- bis 8-gliedrigen gesättigten oder ungesättigten Ring bilden;
R³ Halogen, Cyano, Nitro, Amino, Hydroxy, Carboxy, CHO, CONH₂, SO₂NH₂ oder Z^{b}-R^{b};
R⁴ Wasserstoff, (C₁-C₄)-Alkyl, (C₂-C₄)-Alkenyl oder (C₂-C₄)-Alkinyl;
R⁵ Halogen, Cyano, Nitro, Amino, Hydroxy, Phosphoryl, CHO, CONH₂, SO₂NH₂ oder Z^{c}-R^{c};
R^{a} einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
R^{b}, R^{c} unabhängig voneinander einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
Z^{a} eine divalente Einheit aus der Gruppe O, S, CO, CS, C(O)O, C(O)S, SO, SO₂, NR^{d}, C(O)NR^{d} oder SO₂NR^{d};
Z^{b}, Z^{c} unabhängig voneinander eine direkte Bindung oder eine divalente Einheit aus der Gruppe O, S, CO, CS, C(O)O, C(O)S, SO, SO₂, NR^{d}, SO₂NR^{d} oder C(O)NR^{d};
R^{d} Wasserstoff, (C₁-C₄)-Alkyl oder (C₁-C₄)-Haloalkyl;
n eine ganze Zahl von 0 bis 4
und
m für den Fall, daß X für CH steht, eine ganze Zahl von 0 bis 5, und für den Fall, daß X für N steht, eine ganze Zahl von 0 bis 4
bedeuten,
als Safener zur Verminderung der von Herbiziden an Nutzpflanzen verursachten Schäden.

2. Verwendung von Acylsulfamoylbenzoesäureamiden nach Anspruch 1, worin
R¹ Wasserstoff, 3- bis 8-gliedriges Heterocyclyl mit bis zu drei gleichen oder verschiedenen Heteroatomen aus der Gruppe Stickstoff, Sauerstoff und Schwefel, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl oder Aryl, wobei die sieben letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
R⁵ Halogen, Cyano, Nitro, Amino, Hydroxy, CHO, CONH₂, SO₂NH₂ oder Z^{c}-R^{c};
R^{a} einen (C₂-C₁₂)-Alkylrest, dessen Kohlenstoffkette ein- oder mehrfach durch Sauerstoffatome unterbrochen ist, (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder 3- bis 6-gliedriges Heterocyclyl mit bis zu drei gleichen oder verschiedenen Heteroatomen aus der Gruppe Stickstoff, Sauerstoff und Schwefel, wobei die sieben letztgenannten Reste unabhängig voneinander gegebenenfalls durch einen oder mehrere, gleiche oder verschiedene Reste aus der Gruppe Halogen, Cyano, Nitro, Amino, Hydroxy, Monound Di-[(C₁-C₄)-alkyl]-amino substituiert sind;
R^{b}, R^{c} unabhängig voneinander einen (C₂-C₁₂)-Alkylrest, dessen Kohlenstoffkette ein- oder mehrfach durch Sauerstoffatome unterbrochen ist, (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder 3- bis 6-gliedriges Heterocyclyl mit bis zu drei gleichen oder verschiedenen Heteroatomen aus der Gruppe N, O und S, wobei die sieben letztgenannten Reste unabhängig voneinander gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
Z^{a} eine divalente Einheit aus der Gruppe O, S, CO, CS, C(O)O, C(O)S, SO, SO₂, NR^{d} oder C(O)NR^{d};
Z^{b}, Z^{c} unabhängig voneinander eine direkte Bindung oder eine divalente Einheit aus der Gruppe O, S, CO, CS, C(O)O, C(O)S, SO, SO₂, NR^{d}, SO₂NR^{d} oder C(O)NR^{d} und
R^{d} Wasserstoff, (C₁-C₄)-Alkyl oder (C₁-C₄)-Haloalkyl
bedeuten.

3. Verwendung von Acylsulfamoylbenzoesäureamiden nach Anspruch 1 oder 2, worin
R¹ Wasserstoff, (C₁-C₈)-Alkyl, (C₂-C₆)-Alkenyl, (C₂-C₆)-Alkinyl, (C₃-C₈)-Cycloalkyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sieben letztgenannten Reste gegebenenfalls durch einen oder mehrere, gleiche oder verschiedene Substituenten aus der Gruppe Halogen, Cyano, (C₁-C₆)-Alkoxy, (C₁-C₆)-Alkylthio, (C₁-C₄)-Alkylsulfinyl, (C₁-C₄)-Alkylsulfonyl, (C₂-C₄)-Alkenyloxy, (C₂-C₄)-Alkenylthio, (C₂-C₄)-Alkinyloxy, (C₂-C₄)-Alkinylthio, (C₃-C₆)-Cycloalkyl, (C₅-C₆)-Cycloalkenyl, (C₃-C₆)-Cycloalkoxy, (C₅-C₆)-Cycloalkenyloxy, Mono- und Di-[(C₁-C₄)-alkyl]-amino, [(C₁-C₆)-Alkoxy]-carbonyl, [(C₁-C₆)-Alkylthio]-carbonyl, [(C₁-C₆)-Alkyl]-carbonyl, Phenyl, Phenyl-(C₁-C₄)-alkoxy, 5- bis 6-gliedriges Heterocyclyl mit bis zu drei Heteroatomen aus der Gruppe Stickstoff, Sauerstoff und Schwefel und im Falle cyclischer Reste auch (C₁-C₄)-Alkyl substituiert sind, wobei die zwanzig letztgenannten Reste gegebenenfalls durch einen oder mehrere, gleiche oder verschiedene Substituenten aus der Gruppe Halogen und Cyano und im Falle cyclischer Reste auch (C₁-C₄)-Alkyl substituiert sind;
R³, R⁵ unabhängig voneinander Halogen, Nitro, Amino, Hydroxy, Cyano, SO₂NH₂, (C₁-C₆)-Alkyl, (C₃-C₆)-Cycloalkyl, (C₂-C₆)-Alkenyl, (C₂-C₆)-Alkinyl, (C₁-C₆)-Alkoxy, (C₂-C₆)-Alkenyloxy, (C₂-C₆)-Alkinyloxy, Mono- und Di-[(C₁-C₄)-Alkyl]-aminosulfonyl, (C₁-C₆)-Alkylthio, (C₁-C₈)-Alkylsulfinyl, (C₁-C₆)-Alkylsulfonyl, (C₁-C₆)-Alkoxycarbonyl, (C₁-C₆)-Alkylthiocarbonyl, (C₁-C₆)-Alkylcarbonyl, wobei die fünfzehn letztgenannten Reste gegebenenfalls durch einen oder mehrere, gleiche oder verschiedene Reste aus der Gruppe Halogen, (C₁-C₄)-Haloalkoxy, Cyano, (C₁-C₆)-Alkoxy und im Falle cyclischer Reste auch (C₁-C₄)-Alkyl und (C₁-C₄)-Haloalkyl substituiert sind, bedeuten.

4. Verwendung von Acylsulfamoylbenzoesäureamiden nach einem der Ansprüche 1 bis 3, worin
X CH;
R¹ Wasserstoff, (C₁-C₆)-Alkyl, (C₃-C₆)-Cycloalkyl, (C₂-C₆)-Alkenyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sechs letztgenannten Reste gegebenenfalls durch einen oder mehrere, gleiche oder verschiedene Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-Alkyl und (C₁-C₄)-Haloalkyl substituiert sind;
R² Wasserstoff, (C₁-C₆)-Alkyl, (C₂-C₆)-Alkenyl, (C₂-C₆)-Alkinyl, wobei die drei letztgenannten Reste gegebenenfalls durch einen oder mehrere, gleiche oder verschiedene Substituenten aus der Gruppe Halogen, Hydroxy, (C₁-C₄)-Alkyl, (C₁-C₄)-Alkoxy und (C₁-C₄)-Alkylthio substituiert sind;
R³ Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl;
R⁴ Wasserstoff;
R⁵ Halogen, Nitro, (C₁-C₄)-Alkyl, (C₁-C₄)-Haloalkyl, (C₁-C₄)- Haloalkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl;
n 0, 1 oder 2
und
m 1 oder 2
bedeuten.

5. Verwendung von Acylsulfamoylbenzoesäureamiden nach einem der Ansprüche 1 bis 4, worin
R¹ Wasserstoff, (C₁-C₆)-Alkyl, (C₃-C₆)-Cycloalkyl, wobei die zwei letztgenannten Reste gegebenenfalls durch einen oder mehrere, gleiche oder verschiedene Substituenten aus der Gruppe Halogen, (C₁-C₄)-Alkoxy, (C₁-C₆)-Haloalkoxy und (C₁-C₄)-Alkylthio und im Falle cyclischer Reste auch (C₁-C₄)-Alkyl und (C₁-C₄)-Haloalkyl substituiert sind,
bedeutet.

6. Verwendung von Acylsulfamoylbenzoesäureamiden nach einem der Ansprüche 1 bis 5, worin die Sulfamoylgruppe sich in 4-Position des Phenylringes befindet.

7. Acylsulfamoylbenzoesäureamide der allgemeinen Formel I nach einem der Ansprüche 1 bis 6, mit der Maßgabe, daß
a) R⁵ in der Bedeutung von CH₂OH, CH₂OCH₂CH₃, COOR⁶ oder CONR^{d}R^{c} nicht direkt benachbart zu X gebunden ist, wenn X für N steht,
b) R¹ nicht Phenyl, Benzyl oder 4-Carboxyphenyl bedeutet, wenn X für CH und R², R⁴ und R⁵ jeweils für Wasserstoff stehen.

8. Nutzpflanzenschützende Mittel enthaltend mindestens ein Acylsulfamoylbenzoesäureamid nach Anspruch 7.

9. Nutzpflanzenschützende Mittel enthaltend mindestens ein Acylsulfamoylbenzoesäureamid nach einem der Ansprüche 1 bis 6 und enthaltend mindestens ein Herbizid.

10. Nutzpflanzenschützende Mittel nach Anspruch 9 enthaltend mindestens ein Herbizid aus den Gruppen
A) Phenoxyphenoxy- und Heteroaryloxyphenoxycarbonsäurederivate
B) Sulfonylharnstoffe
C) Chloracetanilide
D) Thiocarbamate
E) Cyclohexandionoxime
F) Imidazolinone
G) Triazolopyrimidinsulfonamidderivate
H) Benzoylcyclohexandione
I) Pyrimidinyloxy-pyridincarbonsäure- bzw. Pyrimidinyloxybenzoesäurederivate
J) S-(N-Aryl-N-alkyl-carbamoylmethyl)-dithiophosphonsäureester

11. Verfahren zum Schutz von Nutzpflanzen vor den phytotoxischen Eigenschaften von Pestiziden, **dadurch gekennzeichnet, daß** nutzpflanzenschützende Mittel gemäß einem der Ansprüche 8 bis 10 in einer Nutzpflanzenkultur verwendet werden.

12. Verfahren zur Herstellung von Acylsulfamoylbenzoesäureamiden nach Anspruch 7, **dadurch gekennzeichnet, daß**
a) eine Verbindung der allgemeinen Formel II 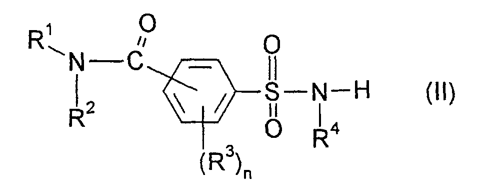 mit einem Acylierungsmittel der allgemeinen Formel III 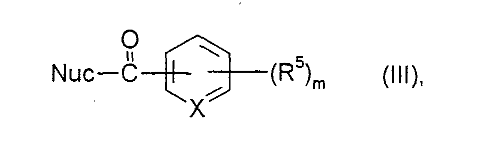 worin Nuc eine Abgangsgruppe ist, umsetzt, oder die Carbonsäure direkt unter Verwendung von Kupplungsreagenzien, wie Dicyclohexylcarbodiimid, mit dem Sulfonamid der Formel II umsetzt,
oder
b) eine Verbindung der allgemeinen Formel IV 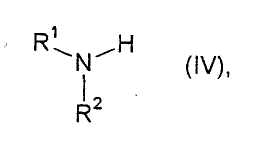 mit aktivierten Carbonsäurederivaten der allgemeinen Formel V 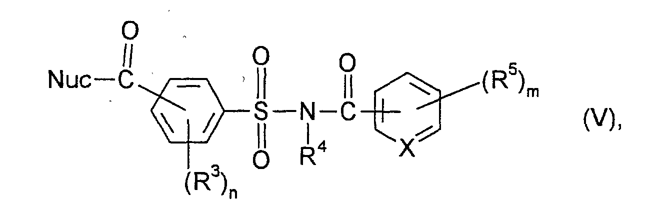
worin Nuc eine Abgangsgruppe bedeutet, umsetzt.

## Claims

1. Use of an acylsulfamoylbenzamide of the formula I, if appropriate also in the form of its salt, 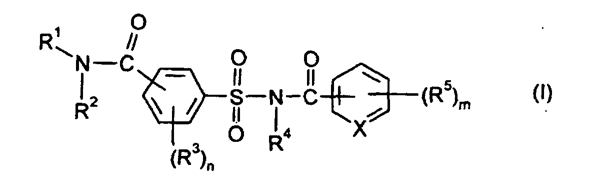 in which
X is CH or N;
R¹ is hydrogen, heterocyclyl or a hydrocarbon radical, where the two last-mentioned radicals are optionally substituted by one or more identical or different radicals selected from the group consisting of halogen, cyano, nitro, amino, hydroxyl, carboxyl, CHO, CONH₂, SO₂NH₂ and Z^{a}-R^{a};
R² is hydrogen, hydroxyl, (C₁-C₆)-alkyl, (C₂-C₆)-alkenyl, (C₂-C₆)-alkynyl, (C₁-C₆)-alkoxy, (C₂-C₆)-alkenyloxy, where the five last-mentioned radicals are optionally substituted by one or more identical or different radicals selected from the group consisting of halogen, hydroxyl, (C₁-C₄)-alkyl, (C₁-C₄)-alkoxy and (C₁-C₄)-alkylthio, or
R¹ and R² together with the linking nitrogen atom form a 3- to 8-membered saturated or unsaturated ring;
R³ is halogen, cyano, nitro, amino, hydroxyl, carboxyl, CHO, CONH₂, SO₂NH₂ or Z^{b}-R^{b};
R⁴ is hydrogen, (C₁-C₄)-alkyl, (C₂-C₄)-alkenyl or (C₂-C₄)-alkynyl;
R⁵ is halogen, cyano, nitro, amino, hydroxyl, phosphoryl, CHO, CONH₂, SO₂NH₂ or Z^{c}-R^{c};
R^{a} is a (C₂-C₂₀)-alkyl radical whose hydrocarbon chain is interrupted once or more than once by oxygen atoms, is heterocyclyl or a hydrocarbon radical, where the two last-mentioned radicals are optionally substituted by one or more identical or different radicals selected from the group consisting of halogen, cyano, nitro, amino, hydroxyl, mono- and di-[(C₁-C₄)-alkyl]amino;
R^{b}, R^{c} independently of one another are a (C₂-C₂₀)-alkyl radical whose hydrocarbon chain is interrupted once or more than once by oxygen atoms, are heterocyclyl or a hydrocarbon radical, where the two last-mentioned radicals are optionally substituted by one or more identical or different radicals selected from the group consisting of halogen, cyano, nitro, amino, hydroxyl, phosphoryl, (C₁-C₄)-haloalkoxy, mono- and di-[(C₁-C₄)-alkyl]amino;
Z^{a} is a divalent unit selected from the group consisting of O, S, CO, CS, C(O)O, C(O)S, SO, SO₂, NRd, C(O)NR^{d} or SO₂NR^{d};
Z^{b}, Z^{c} independently of one another are a direct bond or a divalent unit selected from the group consisting of O, S, CO, CS, C(O)O, C(O)S, SO, SO₂, NR^{d}, SO₂NR^{d} or C(O)NR^{d};
R^{d} is hydrogen, (C₁-C₄)-alkyl or (C₁-C₄)-haloalkyl;
n is an integer from 0 to 4
and
m, in the case that X is CH, is an integer from 0 to 5, and, in the case that X is N, is an integer from 0 to 4,
as safeners for reducing the damage caused by herbicides to useful plants.

2. Use of an acylsulfamoylbenzamide as claimed in claim 1, wherein
R¹ is hydrogen, 3- to 8-membered heterocyclyl having up to three identical or different hetero atoms selected from the group consisting of nitrogen, oxygen and sulfur, (C₁-C₁₂)-alkyl, (C₂-C₈)-alkenyl, (C₂-C₈)-alkynyl, (C₃-C₈)-cycloalkyl, (C₃-C₈)-cycloalkenyl or aryl, where the seven last-mentioned radicals are optionally substituted by one or more identical or different radicals selected from the group consisting of halogen, cyano, nitro, amino, hydroxyl, carboxyl, CHO, CONH₂, SO₂NH₂ and Z^{a}-R^{a};
R⁵ is halogen, cyano, nitro, amino, hydroxyl, CHO, CONH₂. SO₂NH₂ or Z^{c}-R^{c};
R^{a} is a (C₂-C₁₂)-alkyl radical whose hydrocarbon chain is interrupted once or more than once by oxygen atoms, is (C₁-C₈)-alkyl, (C₃-C₆)-cycloalkyl, (C₂-C₈)-alkenyl, (C₃-C₆)-cycloalkenyl, (C₂-C₈)-alkynyl, phenyl or 3- to 6-membered heterocyclyl having up to three identical or different hetero atoms selected from the group consisting of nitrogen, oxygen and sulfur, where the seven last-mentioned radicals independently of one another are optionally substituted by one or more identical or different radicals selected from the group consisting of halogen, cyano, nitro, amino, hydroxyl, mono- and di-[(C₁-C₄)-alkyl]amino;
R^{b}, R^{c} independently of one another are a (C₂-C₁₂)-alkyl radical whose hydrocarbon chain is interrupted once or more than once by oxygen atoms, is (C₁-C₈)-alkyl, (C₃-C₆)cycloalkyl, (C₂-C₈)-alkenyl, (C₃-C₆)-cycloalkenyl, (C₂-C₈)-alkynyl, phenyl or 3- to 6-membered heterocyclyl having up to three identical or different hetero atoms selected from the group consisting of N, O and S, where the seven last-mentioned radicals independently of one another are optionally substituted by one or more identical or different radicals selected from the group consisting of halogen, cyano, nitro, amino, hydroxyl, phosphoryl, (C₁-C₄)-haloalkoxy, mono- and di-((C₁-C₄)-alkyl]amino;
Z^{a} is a divalent unit selected from the group consisting of O, S, CO, CS, C(O)O, C(O)S, SO, SO₂, NR^{d} or C(O)NR^{d};
Z^{b}, Z^{c} independently of one another are a direct bond or a divalent unit selected from the group consisting of O, S, CO, CS, C(O)O, C(O)S, SO, SO₂, NR^{d}, SO₂NR^{d} or C(O)NR^{d}
and
R^{d} is hydrogen, (C₁-C₄)-alkyl or (C₁-C₄)-haloalkyl.

3. Use of an acylsulfamoylbenzamide as claimed in claim 1 or 2, wherein
R¹ is hydrogen, (C₁-C₈)-alkyl, (C₂-C₆)-alkenyl, (C₂-C₆)-alkynyl, (C₃-C₆)-cycloalkyl, (C₅-C₆)-cycloalkenyl, phenyl or 3- to 6-membered heterocyclyl having up to three hetero atoms selected from the group consisting of nitrogen, oxygen and sulfur, where the seven last-mentioned radicals are optionally substituted by one or more identical or different substituents selected from the group consisting of halogen, cyano, (C₁-C₆)-alkoxy, (C₁-C₆)-alkylthio, (C₁-C₄)-alkylsulfinyl, (C₁-C₄)-alkylsulfonyl, (C₂-C₄)-alkenyloxy, (C₂-C₄)-alkenylthio, (C₂-C₄)-alkynyloxy, (C₂-C₄)-alkynylthio, (C₃-C₆)-cycloalkyl, (C₅-C₆)-cycloalkenyl, (C₃-C₆)-cycloalkoxy, (C₅-C₆)-cycloalkenyloxy, mono- and di-[(C₁-C₄)-alkyl]amino, [(C₁-C₆)-alkoxy]carbonyl, [(C₁-C₆)-alkylthio]carbonyl, [(C₁-C₆)-alkyl]carbonyl, phenyl, phenyl-(C₁-C₄)alkoxy, 5- to 6-membered heterocyclyl having up to three hetero atoms selected from the group consisting of nitrogen, oxygen and sulfur and, in the case of cyclic radicals, also by (C₁-C₄)alkyl, where the twenty last-mentioned radicals are optionally substituted by one or more identical or different substituents selected from the group consisting of halogen and cyano and, in the case of cyclic radicals, also by (C₁-C₄)alkyl;
R³, R⁵ independently of one another are halogen, nitro, amino, hydroxyl, cyano, SO₂NH₂, (C₁-C₆)-alkyl, (C₃-C₆)-cycloalkyl, (C₂-C₆)-alkenyl, (C₂-C₆)-alkynyl, (C₁-C₆)-alkoxy, (C₂-C₆)-alkenyloxy, (C₂-C₆)-alkynyloxy, mono- and di- [(C₁-C₄)-alkyl]-aminosulfonyl, (C₁-C₆)-alkylthio, (C₁-C₈)-alkylsulfinyl, (C₁-C₆)-alkylsulfonyl, (C₁-C₆)-alkoxycarbonyl, (C₁-C₆)-alkylthiocarbonyl, (C₁-C₆)-alkylcarbonyl, where the fifteen last-mentioned radicals are optionally substituted by one or more identical or different radicals selected from the group consisting of halogen, (C₁-C₄)-haloalkoxy, cyano, (C₁-C₆)-alkoxy and, in the case of cyclic radicals, also by (C₁-C₄)-alkyl and (C₁-C₄)-haloalkyl.

4. Use of an acylsulfamoylbenzamide as claimed in any of claims 1 to 3, wherein
X is CH,
R¹ is hydrogen, (C₁-C₆)-aIkyl, (C₃-C₆)-cycloalkyl, (C₂-C₆)-alkenyl, (C₅-C₆)-cycloalkenyl, phenyl or 3- to 6-membered heterocyclyl having up to three hetero atoms selected from the group consisting of nitrogen, oxygen and sulfur, where the six last-mentioned radicals are optionally substituted by one or more identical or different substituents selected from the group consisting of halogen, (C₁-C₆)-alkoxy, (C₁-C₆)-haloalkoxy, (C₁-C₂)-alkylsulfinyl, (C₁-C₂)-alkylsulfonyl, (C₃-C₆)-cycloaIkyl, (C₁-C₄)-alkoxycarbonyl, (C₁-C₄)-alkylcarbonyl and phenyl and, in the case of cyclic radicals, also by (C₁-C₄)-alkyl and (C₁-C₄)-haloalkyl;
R² is hydrogen, (C₁-C₆)-alkyl, (C₂-C₆)-alkenyl, (C₂-C₆)-alkynyl, where the three last-mentioned radicals are optionally substituted by one or more identical or different substituents selected from the group consisting of halogen, hydroxyl, (C₁-C₄)-alkyl, (C₁-C₄)-alkoxy and (C₁-C₄)-alkylthio;
R³ is halogen, (C₁-C₄)-haloalkyl, (C₁-C₄)-haloalkoxy, nitro, (C₁-C₄)-alkyl, (C₁-C₄)-alkoxy, (C₁-C₄)-alkylsulfonyl, (C₁-C₄)-alkoxycarbonyl or (C₁-C₄)-alkylcarbonyl;
R⁴ is hydrogen;
R⁵ is halogen, nitro, (C₁-C₄)-alkyl, (C₁-C₄)-haloalkyl, (C₁-C₄)-haloalkoxy, (C₃-C₆)-cycloalkyl, phenyl, (C₁-C₄)-alkoxy, cyano, (C₁-C₄)-alkylthio, (C₁-C₄)-alkylsulfonyl, (C₁-C₄)-alkylsulfonyl, (C₁-C₄)-alkoxycarbonyl or (C₁-C₄)-alkylcarbonyl;
n is 0, 1 or 2 and
m is 1 or 2.

5. Use of an acylsulfamoylbenzamide as claimed in any of claims 1 to 4, wherein
R¹ is hydrogen, (C₁-C₆)-alkyl, (C₃-C₆)-cycloalkyl, where the two last-mentioned radicals are optionally substituted by one or more identical or different substituents selected from the group consisting of halogen, (C₁-C₄)-alkoxy, (C₁-C₆)-haloalkoxy and (C₁-C₄)-alkylthio and, in the case of cyclic radicals, also by (C₁-C₄)-alkyl and (C₁-C₄)-haloalkyl.

6. Use of an acylsulfamoylbenzamide as claimed in any of claims 1 to 5, wherein the sulfamoyl group is in position 4 of the phenyl ring.

7. An acylsulfamoylbenzamide of the formula I as claimed in any of claims 1 to 6 with the proviso that
a) R⁵ in the meaning of CH₂OH, CH₂OCH₂CH₃, COOR^{c} or CONR^{d}R^{c} is not attached directly adjacent to X if X is N,
b) R¹ is not phenyl, benzyl or 4-carboxyphenyl if X is CH and R², R⁴ and R⁵ are each hydrogen.

8. A crop protection composition comprising at least one acylsulfamoylbenzamide as claimed in claim 7.

9. A crop protection composition comprising at least one acylsulfamoylbenzamide as claimed in any of claims 1 to 6 and comprising at least one herbicide.

10. The crop protection composition as claimed in claim 9, comprising at least one herbicide selected from the groups
A) Phenoxyphenoxy- and heteroaryloxyphenoxycarboxylic acid derivatives
B) Sulfonylureas
C) Chloroacetanilides
D) Thiocarbamates
E) Cyclohexanedione oximes
F) Imidazolinones
G) Triazolopyrimidinesulfonamide derivatives
H) Benzoylcyclohexanediones
I) Pyrimidinyloxypyridinecarboxylic acid or pyrimidinyloxybenzoic acid derivatives
J) S-(N-aryl-N-alkylcarbamoylmethyl)dithiophosphonic esters

11. A method for protecting useful plants against the phytotoxic properties of pesticides, which comprises using crop protection compositions as claimed in any of claims 8 to 10 in a crop of useful plants.

12. A process for preparing acylsulfamoylbenzamides as claimed in claim 7, which comprises
a) reacting a compound of the formula II 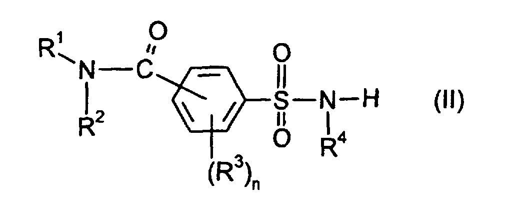 with an acylating agent of the formula III 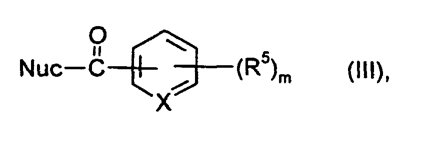 where Nuc is a leaving group, or reacting the carboxylic acid directly with the sulfonamide of the formula II by employing coupling agents, such as dicyclohexylcarbodiimide,
or
b) reacting a compound of the formula IV 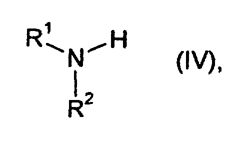 with activated carboxylic acid derivatives of the formula V 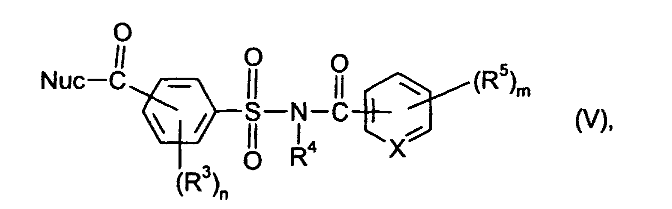 where Nuc is a leaving group.

## Revendications

1. Utilisation d'amides d'acide acylsulfamoylbenzoïque de formule générale I, éventuellement aussi sous leur forme de sel, 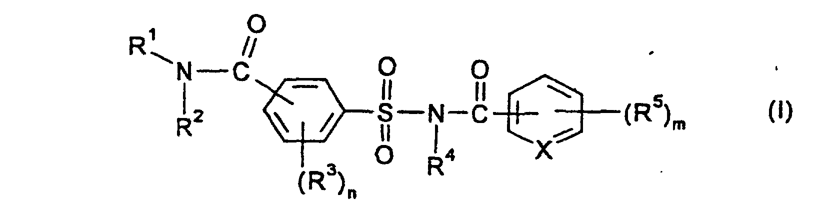 dans laquelle
X représente CH ou N ;
R¹ représente un atome d'hydrogène, un groupe hétérocyclyle ou hydrocarboné, les deux derniers groupes mentionnés étant éventuellement substitués par un ou plusieurs groupes, identiques ou différents, choisis parmi un halogène, un groupe cyano, nitro, amino, hydroxy, carboxy, CHO, CONH₂, SO₂NH₂ et Z^{a}-R^{a};
R² représente un atome d'hydrogène, un groupe hydroxy, alkyle en C₁ à C₆, alcényle en C₂ à C₆, alcynyle en C₂ à C₆, alcoxy en C₁ à C₆, alcényloxy en C₂ à C₆, les cinq derniers groupes mentionnés étant éventuellement substitués par un ou plusieurs groupes, identiques ou différents, choisis parmi un halogène, un groupe hydroxy, alkyle en C₁ à C₄, alcoxy en C₁ à C₄ et alkylthio en C₁ à C₄, ou
R¹ et R² forment ensemble avec l'atome d'azote les portant un cycle saturé ou insaturé de 3 à 8 chaînons ;
R³ représente un halogène, un groupe cyano, nitro, amino, hydroxy, carboxy, CHO, CONH₂, SO₂NH₂ ou Z^{b}-R^{b} ;
R⁴ représente un atome d'hydrogène, un groupe alkyle en C₁ à C₄, alcényle en C₂ à C₄ ou alcynyle en C₂ à C₄ ;
R⁵ représente un halogène, un groupe cyano, nitro, amino, hydroxy, phosphoryle, CHO, CONH₂, SO₂NH₂ ou Z^{c}-R^{c} ; R^{a} représente un groupe alkyle en C₂ à C₂₀ dont la chaîne hydrocarbonée est interrompue une ou plusieurs fois par des atomes d'oxygène, un groupe hétérocyclyle ou hydrocarboné, les deux derniers groupes mentionnés étant éventuellement substitués par un ou plusieurs groupes, identiques ou différents choisis parmi un halogène, un groupe cyano, nitro, amino, hydroxy, mono- et di-[alkyle en C₁ à C₄]-amino ;
R^{b}, R^{c} représentent, indépendamment l'un de l'autre, un groupe alkyle en C₂ à C₂₀, dont la chaîne hydrocarbonée est interrompue une ou plusieurs fois par des atomes d'oxygène, un groupe hétérocyclyle ou hydrocarboné, les deux derniers groupes mentionnés étant éventuellement substitués par un ou plusieurs groupes identiques ou différents choisis parmi un halogène, un groupe cyano, nitro, amino, hydroxy, phosphoryle, halogénoalcoxy en C₁ à C₄, mono- et di-[alkyle en C₁ à C₄]-amino ;
Z^{a} représente un motif divalent choisi parmi O, S, CO, CS, C(O)O, C(O)S, SO, SO₂, NR^{d}, C(O)NR^{d} ou SO₂NR^{d} ;
Z^{b}, Z^{c} représentent, indépendamment l'un de l'autre, une liaison directe ou un motif divalent choisi parmi O, S, CO, CS, C(O)O, C(O)S, SO, SO₂, NR^{d}, SO₂NR^{d} ou C(O)NR^{d} ;
R^{d} représentent un atome d'hydrogène, un groupe alkyle en C₁ à C₄ ou halogénoalkyle en C₁ à C₄ ;
n représente un nombre entier de 0 à 4
et
m dans le cas où X représente CH, représente un nombre entier de 0 à 5, et dans le cas où X représente N, représente un nombre entier de 0 à 4,
comme antidote pour la diminution des dommages provoqués par des herbicides sur ldes plantes utiles.

2. Utilisation d'amides d'acide acylsulfamoylbenzoïque selon la revendication 1, dans laquelle
R¹ représente un atome d'hydrogène, un groupe hétérocyclyle ayant de 3 à 8 chaînons avec jusqu'à trois hétéroatomes identiques ou différents dans le groupe de l'azote, de l'oxygène et du soufre, un groupe alkyle en C₁ à C₁₂, alcényle en C₂ à C₈, alcynyle en C₂ à C₈, cycloalkyle en C₃ à C₈, cycloalcényle en C₃ à C₈ ou aryle, les sept derniers groupes mentionnés étant éventuellement substitués par un ou plusieurs groupes, identiques ou différents, choisis parmi un halogène, un groupe cyano, nitro, amino, hydroxy, carboxy, CHO, CONH₂, SO₂NH₂ et Z^{a}-R^{a} ;
R⁵ représente un halogène, un groupe cyano, nitro, amino, hydroxy, CHO, CONH₂, SO₂NH₂ ou Z^{c}-R^{c} ;
R^{a} représente un groupe alkyle en C₂ à C₁₂ dont la chaîne hydrocarbonée est interrompue une ou plusieurs fois par des atomes d'oxygène, alkyle en C₁ à C₈, cycloalkyle en C₃ à C₆, alcényle en C₂ à C₈, cycloalcényle en C₃ à C₆, alcynyle en C₂ à C₈, phényle ou hétérocyclyle ayant de 3 à 6 chaînons avec jusqu'à trois hétéroatomes, identiques ou différents, dans le groupe de l'azote, de l'oxygène et du soufre, les sept derniers groupes mentionnés étant, indépendamment les uns des autres, éventuellement substitués par un ou plusieurs groupes identiques ou différents choisis parmi un halogène, un groupe cyano, nitro, amino, hydroxy, mono- et di-[alkyle en C₁ à C₄]-amino ;
R^{b}, R^{c} représentent, indépendamment l'un de l'autre, un groupe alkyle en C₂ à C₁₂, dont la chaîne hydrocarbonée est interrompue une ou plusieurs fois par des atomes d'oxygène, alkyle en C₁ à C₈, cycloalkyle en C₃ à C₆, alcényle en C₂ à C₈, cycloalcényle en C₃ à C₆, alcynyle en C₂ à C₈, phényle ou hétérocyclyle ayant de 3 à 6 chaînons avec jusqu'à trois hétéroatomes, identiques ou différents, dans le groupe N, O ou S, les sept derniers groupes mentionnés étant, indépendamment les uns des autres, éventuellement substitués par un ou plusieurs groupes identiques ou différents choisis parmi un halogène, un groupe cyano, nitro, amino, hydroxy, phosphoryle, halogénoalcoxy en C₁ à C₄, mono- et di-[alkyle en C₁ à C₄]-amino ;
Z^{a} représente un motif divalent choisi parmi O, S, CO, CS, C(O)O, C(O)S, SO, SO₂, NR^{d}, C(O)NR^{d} ;
Z^{b}, Z^{c} représentent, indépendamment l'un de l'autre, une liaison directe ou un motif divalent choisi parmi O, S, CO, CS, C(O)O, C(O)S, SO, SO₂, NR^{d}, SO₂NR^{d} ou C(O)NR et
R^{d} représente un atome d'hydrogène, un groupe alkyle en C₁ à C₄ ou halogénoalkyle en C₁ à C₄.

3. Utilisation d'amides d'acide acylsulfamoylbenzoïque selon la revendication 1, dans laquelle
R¹ représente un atome d'hydrogène, un groupe alkyle en C₁ à C₈, alcényle en C₂ à C₆, alcynyle en C₂ à C₆, cycloalkyle en C₃ à C₆, cycloalcényle en C₅ à C₆, phényle ou hétérocyclyle ayant de 3 à 6 chaînons avec jusqu'à trois hétéroatomes dans le groupe de l'azote, de l'oxygène et du soufre, les sept derniers groupes mentionnés étant éventuellement substitués par un ou plusieurs groupes, identiques ou différents, choisis parmi un halogène, un groupe cyano, alcoxy en C₁ à C₆, alkylthio en C₁ à C₆, alkylsulfinyle en C₁ à C₄, alkylsulfonyle en C₁ à C₄, alcényloxy en C₂ à C₄, alcénylthio en C₂ à C₄, alcynyloxy en C₂ à C₄, alcynylthio en C₂ à C₄, cycloalkyle en C₃ à C₆, cycloalcényle en C₅ à C₆, cycloalcoxy en C₃ à C₆, cycloalcényloxy en C₅ à C₆, mono- et di-[alkyle en C₁ à C₄]-amino, [alcoxy en C₁ à C₆]-carbonyle, [alkylthio en C₁ à C₆]-carbonyle, [alkyle en C₁ à C₆]-carbonyle, phényle, phényl-(alcoxy en C₁ à C₄), hétérocyclyle ayant 5 à 6 chaînons avec jusqu'à trois hétéroatomes dans le groupe de l'azote, de l'oxygène et du soufre et dans le cas de groupes cycliques étant aussi substitués par un alkyle en C₁ à C₄, les vingt derniers groupes mentionnés étant, indépendamment les uns des autres, éventuellement substitués par un ou plusieurs substituants identiques ou différents choisis parmi un halogène et un groupe cyano et dans le cas de groupes cycliques aussi substitués par un alkyle en C₁ à C₄ ;
R³, R⁵ représentent, indépendamment l'un de l'autre, un halogène, un groupe nitro, amino, hydroxy, cyano, SO₂NH₂, alkyle en C₁ à C₆, cycloalkyle en C₃ à C₆, alcényle en C₂ à C₆, alcynyle en C₂ à C₆, alcoxy en C₁ à C₆, alcényloxy en C₂ à C₆, alcynyloxy en C₂ à C₆, mono- et di-[alkyle en C₁ à C₄]-aminosulfonyle, alkylthio en C₁ à C₆, alkylsulfinyle en C₁ à C₈, alkylsulfonyle en C₁ à C₆, alcoxycarbonyle en C₁ à C₆, alkylthiocarbonyle en C₁ à C₆, alkylthiocarbonyle en C₁ à C₆, alkylcarbonyle en C₁ à C₆, les quinze derniers groupes mentionnés étant éventuellement substitués par un ou plusieurs substituants identiques ou différents choisis parmi un halogène, un groupe halogénoalcoxy en C₁ à C_{4,} cyano, alcoxy en C₁ à C₆ et dans le cas de groupes cycliques étant aussi substitués par un groupe alkyle en C₁ à C₄ et halogénoalkyle en C₁ à C₄.

4. Utilisation d'amides d'acide acylsulfamoylbenzoïque selon l'une des revendications 1 à 3, dans laquelle
X représente CH ;
R¹ représente un atome d'hydrogène, un groupe alkyle en C₁ à C₆, cycloalkyle en C₃ à C₆, alcényle en C₂ à C₆, cycloalcényle en C₅ à C₆, phényle ou hétérocyclyle ayant de 3 à 6 chaînons avec jusqu'à trois hétéroatomes dans le groupe de l'azote, de l'oxygène et du soufre, les six derniers groupes mentionnés étant éventuellement substitués par un ou plusieurs substituants, identiques ou différents, choisis parmi un halogène, un groupe alcoxy en C₁ à C₆, halogénoalcoxy en C₁ à C₆, alkylsulfinyle en C₁ à C₂, alkylsulfonyle en C₁ à C₂, cycloalkyle en C₃ à C₆, alcoxycarbonyle en C₁ à C₄, alkylcarbonyle en C₁ à C₄ et phényle et dans le cas de groupes cycliques étant aussi substitués par un groupe alkyle en C₁ à C₄ et halogéno-alkyle en C₁ à C₄ ;
R² représente un atome d'hydrogène, un groupe alkyle en C₁ à C₆, alcényle en C₂ à C₆, alcynyle en C₂ à C₆, les trois derniers groupes mentionnés étant éventuellement substitués par un ou plusieurs substituants, identiques ou différents, choisis parmi un halogène, un groupe hydroxy, alkyle en C₁ à C₄, alcoxy en C₁ à C₄ et alkylthio en C₁ à C₄ ;
R³ représente un halogène, un groupe halogénoalkyle en C₁ à C₄, halogénoalcoxy en C₁ à C₄, nitro, alkyle en C₁ à C₄, alcoxy en C₁ à C₄, alkylsulfonyle en C₁ à C₄, alcoxycarbonyle en C₁ à C₄ ou alkylcarbonyle en C₁ à C₄,
R⁴ représente un atome d'hydrogène ;
R⁵ représente un halogène, un groupe nitro, alkyle en C₁ à C₄, halogénoalkyle en C₁ à C₄, halogénoalcoxy en C₁ à C₄, cycloalkyle en C₃ à C₆, phényle, alcoxy en C₁ à C₄, cyano, alkylthio en C₁ à C₄, alkylsulfinyle en C₁ à C₄, alkylsulfonyle en C₁ à C₄, alcoxy en C₁ à C₄-carbonyle ou alkyle en C₁ à C₄-carbonyle ;
n représente 0, 1 ou 2
et
m représente 1 ou 2.

5. Utilisation d'amides d'acide acylsulfamoylbenzoïque selon l'une des revendications 1 à 4, dans laquelle
R¹ représente un atome d'hydrogène, un groupe alkyle en C₁ à C₆, cycloalkyle en C₃ à C₆, les deux derniers groupes mentionnés étant éventuellement substitués par un ou plusieurs substituants, identiques ou différents, choisis parmi un halogène, un groupe alcoxy en C₁ à C₄, halogénoalcoxy en C₁ à C₆ et alkylthio en C₁ à C₄ et dans le cas de groupes cycliques aussi un groupe alkyle en C₁ à C₄ et halogénoalkyle en C₁ à C₄.

6. Utilisation d'amides d'acide acylsulfamoylbenzoïque selon l'une des revendications 1 à 5, dans laquelle le groupe sulfamoyle se trouve en position 4 du cycle phényle.

7. Amides d'acide acylsulfamoylbenzoïque de formule générale I selon l'une des revendications 1 à 6, avec la condition que
a) R⁵ au sens de CH₂OH, CH₂OCH₂CH₃, COOR^{c} ou CONR^{d}R^{c} ne soit pas lié au voisinage direct de X lorsque X représente N,
b) R¹ ne représente pas un groupe phényle, benzyle ou 4-carboxyphényle lorsque X représente CH et R², R⁴ et R⁵ représentant respectivement un atome d'hydrogène.

8. Agents de protection des plantes utiles contenant au moins un amide d'acide acylsulfamoylbenzoïque selon la revendication 7.

9. Agents de protection des plantes utiles contenant au moins un amide d'acide acylsulfamoylbenzoïque selon l'une des revendications 1 à 6 et contenant au moins un herbicide.

10. Agents de protection des plantes utiles selon la revendication 9 contenant au moins un herbicide choisi parmi
A) les dérivés d'acide phénoxyphénoxy- et hétéroaryloxyphénoxycarboxylique,
B) les sulfonylurées,
C) les chloroacétanilides,
D) les thiocarbamates,
E) les cyclohexanedionoximes,
F) les imidazolinones,
G) les dérivés de triazolopyrimidinesulfonamide,
H) les benzoylcyclohexanediones,
I) les dérivés d'acide pyrimidinyloxy-pyridinecarboxylique ou d'acide pyrimidinyloxybenzoïque,
J) les esters d'acide S-(N-aryl-N-alkyl-carbamoylméthyl)-dithiophosphonique.

11. Procédé pour la protection de plantes utiles contre les caractéristiques phytotoxiques de pesticides, **caractérisé en ce qu'**on utilise des agents de protection des plantes utiles selon une des revendications 8 à 10 dans une culture de plantes utiles.

12. Procédé pour la préparation d'amides d'acide acylsulfamoylbenzoïque selon la revendication 7, **caractérisé en ce qu'**on met à réagir
a) un composé de formule générale II 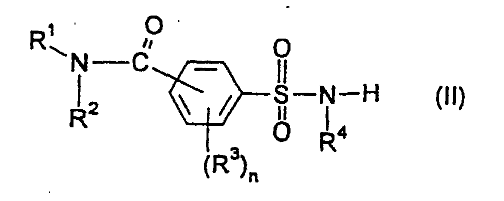 avec un agent d'acylation de formule générale III 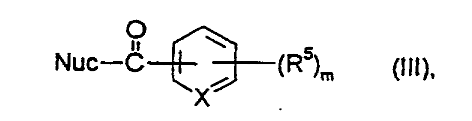 dans laquelle Nuc est un groupe partant, ou on met à réagir l'acide carboxylique directement en utilisant des réactifs de couplage, comme le dicyclohexylcarbodiimide, avec le sulfonamide de formule II,
ou on met à réagir
b) un composé de formule générale IV 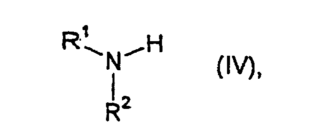 avec des dérivés d'acide carboxylique activés de formule générale V 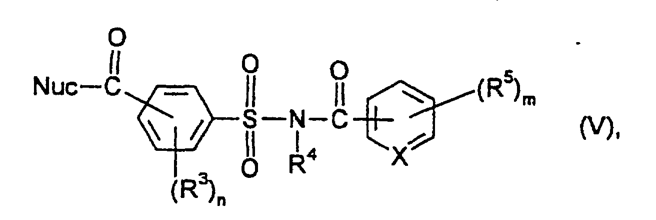 dans laquelle Nuc représente un groupe partant.
